(19) Europäisches Patentamt
European Patent Office
Office européen des brevets

(11) Veröffentlichungsnummer: **0 268 178**
**A1**

(12) # EUROPÄISCHE PATENTANMELDUNG

(21) Anmeldenummer: 87116512.2

(22) Anmeldetag: 09.11.87

(51) Int. Cl.4: **C07D 487/04**, A61K 31/415, //(C07D487/04,235:00,209:00)

(30) Priorität: 18.11.86 DE 3639466

(43) Veröffentlichungstag der Anmeldung:
**25.05.88 Patentblatt 88/21**

(84) Benannte Vertragsstaaten:
**AT BE CH DE ES FR GB GR IT LI LU NL SE**

(71) Anmelder: **Dr. Karl Thomae GmbH**
**Postfach 1755**
**D-7950 Biberach (Riss)(DE)**

(72) Erfinder: **Narr, Berthold, Dr.Dipl.-Chem.**
**Obere Au 5**
**D-7950 Biberach 1(DE)**
Erfinder: **Hauel, Norbert, Dr.Dipl.-Chem.**
**Stresemannstrasse 40**
**D-7950 Biberach 1(DE)**
Erfinder: **Noll, Klaus, Dr. Dipl.-Chem.**
**Im Schönblick 3**
**D-7951 Warthausen 1(DE)**
Erfinder: **Heider, Joachim, Dr. Dipl.-Chem.**
**Am Hang 3**
**D-7951 Warthausen 1(DE)**
Erfinder: **Psiorz, Manfred, Dr. Dipl.-Chem.**
**Riedlinger Str. 35**
**D-7950 Biberach1(DE)**
Erfinder: **Bomhard, Andreas, Dr. Dipl.-Chem.**
**Dinglingerstr. 9**
**D-7950 Biberach 1(DE)**
Erfinder: **van Meel, Jacques, Dr.**
**Amriswilstrasse 7**
**D-7950 Biberach 1(DE)**
Erfinder: **Diederen, Willi, Dr.**
**Haldenstrasse 1a**
**D-7950 Biberach 1(DE)**

(54) **Neue Pyrrolo-benzimidazole, diese Verbindungen enthaltende Arzneimittel und Verfahren zu ihrer Herstellung.**

(57) Die Erfindung betrifft neue Pyrrolo-benzimidazole der allgemeinen Formel

EP 0 268 178 A1

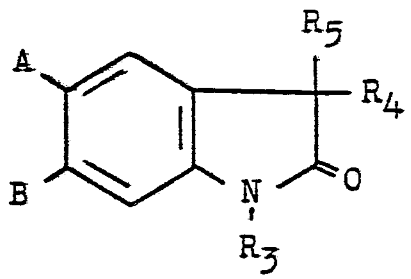

(I)

in der

A und B zusammen mit den beiden dazwischenliegenden Kohlenstoffatomen eine Imidazogruppe der Formel

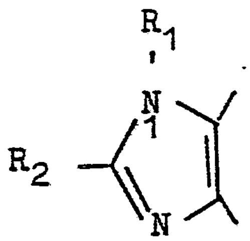

oder

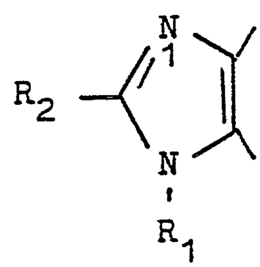

darstellt, wobei

$R_1$ ein Wasserstoffatom oder eine gegebenenfalls durch eine Phenylgruppe substituierte Alkylgruppe und

$R_2$ ein Wasserstoffatom, eine gegebenenfalls durch eine Phenyl-, Pyridyl-oder Thienylgruppe substituierte Alkylgruppe, eine Vinylengruppe, die endständig durch eine Phenyl-oder Pyridylgruppe substituiert ist, eine gegebenenfalls durch ein Halogenatom, eine Hydroxy-, Alkoxy-, Mercapto-, Alkylmercapto-, Alkylsulfinyl-, Alkylsulfonyl-, Cyano-, Amino-, Alkylamino-, Dialkylamino-, Alkanoylamino-oder Ni trogruppe substituierte Phenylgruppe, wobei eine Hydroxyphenylgruppe zusätzlich durch eine oder zwei Alkylgruppen oder eine Aminophenylgruppe durch ein oder zwei Halogenatome substituiert sein kann, eine durch Halogenatome, Hydroxy-, Alkoxy-, Mercapto-, Alkylmercapto-, Alkylsulfinyl-oder Alkylsulfonylgruppen disubstituierte Phenylgruppe, wobei die Substituenten des Phenylkerns gleich oder verschieden sein können, einen über ein Kohlenstoffatom gebundenen 5-oder 6-gliedrigen heteroaromatischen Ring, welcher ein Sauerstoff-, Schwefel-oder Stickstoffatom, zwei oder drei Stickstoffatome oder ein Stickstoffatom und ein Sauerstoff- oder Schwefelatom enthält, an den zusätzlich über zwei benachbarte Kohlenstoffatome eine oder zwei 1,4-Butadienylgruppen gebunden sein können, wobei in diesem Falle auch die Bindung über einen ankondensierten Phenylring erfolgen kann und im Falle der Pyridine diese zusätzlich durch eine Amino-, Alkylamino-, Dialkylamino-, Alkanoylamino-oder Morpholinogruppe oder durch zwei Halogenatome oder durch ein Halogenatom und eine Amino-oder Morpholinogruppe substituiert und im Falle der Chinoline zusätzlich durch ein Halogenatom, eine Alkoxy-oder Phenylgruppe mono-oder disubstituiert, wobei die Substituenten gleich oder verschieden sein können, sein können, einen über einen Kohlenstoffatom gebundenen 5-bis 7-gliedrigen gesättigten Imino-, N-Alkyl-imino-oder N-Alkanoylimino-alkylenring, eine 1,2,3,4-Tetrahydro-chinolyl-oder 5,6,7,8-Tetrahydro-chinolylgruppe darstellen,

$R_3$ eine gegebenenfalls durch eine Phenylgruppe substitu ierte Alkylgruppe, eine Alkylgruppe oder eine ab Position 2 durch eine Hydroxy-, Alkoxy-oder Pyridiniumgruppe substituierte Alkylgruppe,

$R_4$ und $R_5$, die gleich oder verschieden sein können, Wasserstoffatome, Alkylgruppen oder Cycloalkylgruppen bedeuten, und deren Säureadditionssalze, insbesondere für die pharma zeutische Anwendung deren physiologisch verträgliche Säureadditionssalze mit anorganischen oder organischen Säuren, welche wertvolle pharmakologische Eigenschaften aufweisen, insbesondere bei einer leichten Beeinflussung des Blutdruckes eine positiv inotrope Wirkung und eine antithrombotische Wirkung.

Die neuen Verbindungen lassen sich nach bekannten Methoden herstellen.

**Neue Pyrrolo-benzimidazole, diese Verbindungen enthaltende Arzneimittel und Verfahren zu ihrer Herstellung**

In der DE-Al-3.501.497 werden bereits in 5-Stellung unsubstituierte Pyrrolo-benzimidazole und diese Verbindungen enthaltende Arzneimittel zur Behandlung von Herz-und Kreislaufkrankheiten beschrieben

Es wurde nun gefunden, daß die neuen in 5-Stellung substituierten Pyrrolo-benzimidazole der Formel

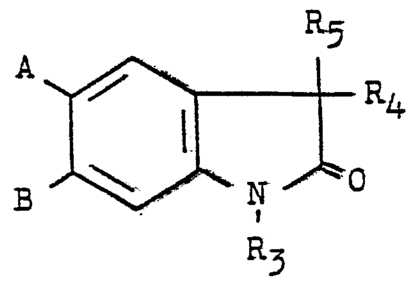

(I)

in der

A und B zusammen mit den beiden dazwischenliegenden Kohlenstoffatomen eine Imidazogruppe der Formel

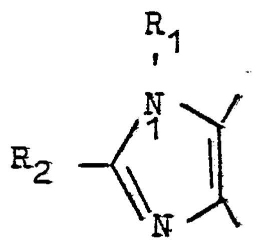

oder

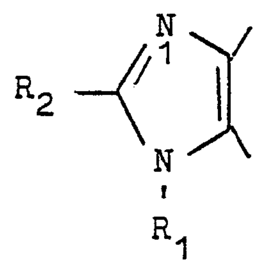

darstellt, und deren Säureadditionssalze, insbesondere für die pharmazeutische Anwendung deren physiologisch verträglich Säureaditionssalze mit anorganischen oder organischen Säuren, überlegene pharmakologische Eigenschaften aufweisen, insbesondere überlegene antithrombotische und cardiovasculäre Eigenschaften.

Gegenstand der vorliegenden Erfindung sind somit die neuen in 5-Stellung subtituierten Pyrrolo-benzimidazole der obigen Formel I, deren Säureadditionssalze, insbesondere für die pharmazeutische Anwendung deren physiologisch verträgliche Säureadditionssalze, diese Verbindungen enthaltende Arzneimittel und Verfahren zu ihrer Herstellung.

In der obigen Formel I bedeutet

$R_1$ ein Wasserstoffatom oder eine gegebenenfalls durch eine Phenylgruppe substituierte Alkylgruppe mit 1 bis 3 Kohlenstoffatomen,

$R_2$ ein Wasserstoffatom, eine gegebenenfalls durch eine Phenyl-, Pyridyl-oder Thienylgruppe substituierte Alkylgruppe, eine Vinylengruppe, die endständig durch eine Phenyl-oder Pyridylgruppe substituiert ist, eine gegebenenfalls durch ein Halogenatom, eine Hydroxy-, Alkoxy-, Mercapto-, Alkylmercapto-, Alkylsulfinyl-, Alkylsulfonyl-, Cyano-, Amino-, Alkylamino-, Dialkylamino-, Alkanoylamino-oder Nitrogruppe substituierte Phenylgruppe, wobei eine Hydroxyphenylgruppe zusätzlich durch eine oder zwei Alkylgruppen mit jeweils 1 bis 4 Kohlenstoffatomen oder eine Aminophenylgruppe durch ein oder zwei Halogenatome substituiert sein kann, eine durch Halogenatome, Hydroxy-, Alkoxy-, Mercapto-, Alkylmercapto-, Alkylsulfinyl-oder Alkylsulfonylgruppen disubstituierte Phenylgruppe, wobei die Substituenten des Phenylkerns gleich oder verschieden sein können, einen über ein Kohlenstoffatom gebundenen 5-oder 6-gliedrigen heteroaroma tischen Ring, welcher ein Sauerstoff-, Schwefel-oder Stickstoffatom, zwei oder drei Stickstoffatome oder ein Stickstoffatom und ein Sauerstoff-oder Schwefelatom enthält, an den zusätzlich über zwei benachbarte Kohlenstoffatome eine oder zwei 1,4-Butadienylgruppen gebunden sein können, wobei in diesem Falle auch die

Bindung über einen ankondensierten Phenylring erfolgen kann und im Falle der Pyridine diese zusätzlich durch eine Amino-, Alkylamino-, Dialkylamino , Alkanoylamino-oder Morpholinogruppe oder durch zwei Halogenatome oder durch ein Halogenatom und eine Amino-oder Morpholinogruppe substituiert und im Falle der Chinoline zusätzlich durch ein Halogenatom, eine Alkoxy-oder Phenylgruppe monooder disubstituiert, wobei die Substituenten gleich oder verschieden sein können, sein können, einen über einen Kohlenstoffatom gebundenen 5-bis 7-gliedrigen gesättigten Imino-, N-Alkyl-imino-oder N-Alkanoyl-iminoalkylenring, eine 5,6,7.8-Tetrahydro-chinolylgruppe oder eine gegebenenfalls am N-Atom durch eine Alkanoylgruppe substituierte 1,2,3,4-Tetrahydro-chinolylgruppe, wobei alle bei der Definition des Restes $R_2$ vorstehend erwähnten Ringe durch eine Alkylgruppe substituiert sein können und der Alkylteil aller vorstehend erwähnten Gruppen, sofern nichts anderes erwähnt wird, jeweils 1 bis 3 Kohlenstoffatome und der Alkanoylteil zwei oder drei Kohlenstoffatome enthalten kann,

$R_3$ eine gegebenenfalls durch eine Phenylgruppe substituierte Alkylgruppe, eine Alkylgruppe mit 4 bis 6 Kohlenstoffatomen oder eine in 2-oder 3-Position durch eine Hydroxy-, Alkoxy-oder Pyridiniumgruppe substituierte Alkylgruppe, wobei der Alkylteil jeweils 1 bis 3 Kohlenstoffatome enthalten kann,

$R_4$ und $R_5$, die gleich oder verschieden sein können, Wasserstoffatome, Alkylgruppen mit 1 bis 3 Kohlenstoffatomen oder Cycloalkylgruppen mit 3 bis 7 Kohlenstoffatomen.

Für die bei der Definition der Reste $R_1$ bis $R_5$ eingangs erwähnten Bedeutung kommt beispielsweise

für $R_1$ die Bedeutung des Wasserstoffatoms, der Methyl-, Äthyl-, n-Propyl-, Isopropyl-, Benzyl-, 1-Phenyläthyl-, 2-Phenyläthyl-oder 3-Phenylpropylgruppe,

für $R_2$ die des Wasserstoffatoms, der Methyl-, Äthyl-, n-Propyl-, Isopropyl-, Benzyl-, 1-Phenylethyl-, 2-Phenylethyl-, 3-Phenylpropyl-, Pyridylmethyl-, Methylpyridylmethyl-, 1-Pyridylethyl-, 2-Pyridylethyl-, 2-Phenylethenyl-, 2-Pyridylethenyl-, Phenyl-, Fluorphenyl-, Difluorphenyl-, Chlorphenyl-, Dichlorphenyl-, Bromphenyl-, Dibromphenyl-, Hydroxyphenyl-, Dihydroxyphenyl-, Methoxyphenyl-, Dimethoxyphenyl-, Mercaptophenyl-, Bis(Mercapto)-phenyl-, Methylmercaptophenyl-, Bis(Methylmercapto)-phenyl-, Methylsulfinylphenyl-, Bis(Methylsulfinyl)-phenyl-, Methylsulfonylphenyl-, Bis(Methylsulfonyl)-phenyl-, Fluorhydroxyphenyl-, Fluor-methoxyphenyl-, Fluor-mercaptophenyl-, Fluor-methylmercaptophenyl-, Chlorhydroxyphenyl-, Chlor-methoxyphenyl-, Chlor-mercaptophenyl-, Chlor-methylmercaptophenyl-, Bromhydroxyphenyl-, Brom-methoxyphenyl-, Brom-mercaptophenyl-, Brom-methylmercaptophenyl-, Hydroxymethoxyphenyl-, Hydroxy-mercaptophenyl-, Hydroxy-methylmercaptophenyl-, Methoxy-mercaptophenyl-, Methoxy-methylmercaptophenyl-, Methoxy-methylsulfinylphenyl-, Methoxy-methylsulfonylphenyl-, Cyanophenyl-, Aminophenyl-, Methylaminophenyl-, Dimethylaminophenyl-, Acetylaminophenyl-, Aminodichlorphenyl-, Amino-dibromphenyl-, Nitrophenyl-, 4-Hydroxy-3,5-di-tert.butyl-phenyl-, Pyridyl-, Methylpyridyl-, Ethylpyridyl-, Isopropylpyridyl-, 2-Aminopyridyl-5-, 2-Formylamino-pyridyl-5-, 2-Acetylamino-pyridyl-5-, 2-Propionylamino-pyridyl-5-, 2,6-Dichlor-pyridyl-4-, 2-Chlor-6-amino-pyridyl-4-, 2-Chlor-6-morpholino-pyridyl-4-, 2,6-Dichlor-pyridyl-3-, 2-Chlor-6-morpholino-pyridyl-3-, Pyrrolyl-2-, Pyrrolyl-3-, N-Methyl-pyrrolyl-2-, N-Ethylpyrrolyl-2-, N-Methyl-pyrrolyl-3-, N-Ethyl-pyrrolyl-3-, Furyl-2-, Furyl-3-, 5-Methyl-furyl-2-, 2-Methyl-furyl-3-, 5-Methyl-furyl-3-, Pyrazinyl-2-, Pyrimidyl-2-, Pyrimidyl-4-, Pyrimidyl-5-, Pyridazinyl-4-, Benzo[b]furyl-2-, Benzo[b] furyl-3-, 7-Methyl-benzo[b]furyl-3-, Thienyl-2-, Thienyl-3-, Thienyl-2-methyl-, Thienyl-3-methyl-, 5-Methyl-thienyl-2-, 2-Methyl-thienyl-3-, 3-Methyl-thienyl-2-, Benzo[b]thienyl-2-, Benzo[b]thienyl-3-, Benzo[b]thienyl-4-, Benzo[b]thienyl-5-, Benzo[b]thienyl-6-, Benzo[b]thienyl-7-, Pyrazolyl-3-, Imidazolyl-2-, Imidazolyl-4(5)-, 1-Methyl-imidazolyl-4-, Benzo[d]imidazolyl-2-, Oxazolyl-2-, Oxazolyl-4-, Oxazolyl-5-, 4-Methyl-oxazolyl-5-, Isoxazolyl-3-, 3-Methylisoxazolyl-5-, 5-Methyl-isoxazolyl-3-, Thiazolyl-2-, Thiazolyl-5-, 4-Methyl-thiazolyl-5-, Pyrazolyl-4-, Triazolyl-3-, Benzo[d]oxazolyl-2-, Benzo[d]-isoxazolyl-3-, Benzo[d]thiazolyl-2-, Benzo[d]isothiazolyl-3-, Benzo[d]pyrazolyl-3-, Indolyl-2-, Indolyl-3-, 5-Methyl-indolyl-3-, 7-Methyl-indolyl-3-, N-Methyl-indolyl-3-, Chinolyl-2-, Chinolyl-4-, Isochinolyl-1-, 2-Methyl-chinolyl-4-, 7-Methyl-chinolyl-2-, 2-Phenyl-chinolyl-4-, 2-Chlor-6-methoxy-chinolyl-4-, 4-Chlo-r7-trifluormethyl-chinolyl-3-, 1,2,3,4-Tetrahydro-chinolyl-2-, 1,2,3,4-Tetrahydro-chinolyl-4-, 5,6,7,8-Tetrahydro-chinolyl-2-, 5,6,7,8-Tetrahydro-chinolyl-4-, Acridinyl-6-, Pyrrolidinyl-3-, Piperidinyl-4-, Hexahydro-azepinyl-4-, 1-Methylpiperidinyl-4-oder 1-Acetyl-piperidinyl-4-gruppe,

für $R_3$ die der Methyl-, Ethyl-, n-Propyl-, Isopropyl-, n-Butyl-, Isobutyl-, tert.Butyl-, n-Pentyl-, 1-Methyl-n-butyl-, 2-Methyl-n-butyl-, 3-Methyl-n-butyl-, 1-Äthyl-n-propyl-, tert.Pentyl-, n-Hexyl-, 2-Hydroxy-äthyl-, 2-Hydroxyn-propyl-, 3-Hydroxy-n-propyl-, 1-Methyl-2-hydroxy-äthyl-, 2-Methoxy-äthyl-, 2-Äthoxy-äthyl-, 2-Methoxy-n-propyl-, 2-n-Propoxy-n-propyl-, 3-Methoxy-n-propyl-, 3-Äthoxy-n-propyl-, 1-Methyl-2-methoxy-äthyl-, 1-Methyl-2-isopropoxyäthyl-, 2-Pyridinium-äthyl-, 3-Pyridinium-n-propyl-, Benzyl-, 1-Phenyläthyl-, 2-Phenyläthyl-oder 3-Phenylpropylgruppe

für $R_4$ und $R_5$ jeweils die des Wasserstoffatoms, der Methyl-, Äthyl-, n-Propyl-, Isopropyl-, Cyclopropyl-, Cyclobutyl-, Cyclopentyl-, Cyclohexyl-oder Cycloheptylgruppe in Betracht.

Beispielsweise seien folgende Verbindungen erwähnt, die unter die vorstehend erwähnte Formel I fallen und nicht in den Beispielen explicit beschrieben werden:

5-Methyl-2-(2-pyridyl)-6,7-dihydro-3H,5H-pyrrolo[2,3-f]benzimidazol-6-on,

5-Methyl-2-(4-methoxy-phenyl)-6,7-dihydro-3H,5H-pyrrolo[2,3-f]benzimidazol-6-on,

5-Methyl-2-(4-hydroxy-phenyl)-6,7-dihydro-3H,5H-pyrrolo[2,3-f]benzimidazol-6-on,

5-Äthyl-2-(4-pyridyl)-6,7-dihydro-3H,5H-pyrrolo[2,3-f]benzimidazol-6-on,

5-Äthyl-2-(3-pyridyl)-6,7-dihydro-3H,5H-pyrrolo[2,3-f]benzimidazol-6-on,

5-Äthyl-2-(2-pyridyl)-6,7-dihydro-3H,5H-pyrrolo[2,3-f]benzimidazol-6-on,

5-Äthyl-2-(4-methoxy-phenyl)-6,7-dihydro-3H,5H-pyrrolo[2,3-f]benzimidazol-6-on,

5-Äthyl-2-(4-hydroxy-phenyl)-6,7-dihydro-3H,5H-pyrrolo[2,3-f]benzimidazol-6-on,

5-(2-Methoxy-äthyl)-2-(4-pyridyl)-6,7-dihydro-3H,5H-pyrrolo[2,3-f]benzimidazol-6-on,

5-(2-methoxy-äthyl)-2-(3-pyridyl)-6,7-dihydro-3H,5H-pyrrolo[2,3-f]benzimidazol-6-on,

5-(2-Methoxy-äthyl)-2-(2-pyridyl)-6,7-dihydro-3H,5H-pyrrolo[2,3-f]benzimidazol-6-on,

5-(2-Methoxy-äthyl)-2-(4-methoxy-phenyl)-6,7-dihydro-3H,5H-pyrrolo[2,3-f]benzimidazol-6-on,

5-(2-Methoxy-äthyl)-2-(4-hydroxy-phenyl)-6,7-dihydro-3H,5H-pyrrolo[2,3-f]benzimidazol-6-on,

5-(2-Hydroxy-äthyl)-2-(4-pyridyl)-6,7-dihydro-3H,5H-pyrrolo[2,3-f]benzimidazol-6-on,

5-(2-Hydroxy-äthyl)-2-(3-pyridyl)-6,7-dihydro-3H,5H-pyrrolo[2,3-f]benzimidazol-6-on,

5-(2-Hydroxy-äthyl)-2-(2-pyridyl)-6,7-dihydro-3H,5H-pyrrolo[2,3-f]benzimidazol-6-on,

5-(2-Hydroxy-äthyl)-2-(4-methoxy-phenyl)-6,7-dihydro-3H,5H-pyrrolo[2,3-f]benzimidazol-6-on,

5-(2-Hydroxy-äthyl)-2-(4-hydroxy-phenyl)-6,7-dihydro-3H,5H-pyrrolo[2,3-f]benzimidazol-6-on,

5,7-Dimethyl-2-(4-pyridyl)-6,7-dihydro-3H,5H-pyrrolo[2,3-f]benzimidazol-6-on,

5,7-Dimethyl-2-(3-pyridyl)-6,7-dihydro-3H,5H-pyrrolo[2,3-f]benzimidazol-6-on,

5,7-Dimethyl-2-(2-pyridyl)-6,7-dihydro-3H,5H-pyrrolo[2,3-f]benzimidazol-6-on,

5,7-Dimethyl-2-(4-methoxy-phenyl)-6,7-dihydro-3H,5H-pyrrolo[2,3-f]benzimidazol-6-on,

5,7-Dimethyl-2-(4-hydroxy-phenyl)-6,7-dihydro-3H,5H-pyrrolo[2,3-f]benzimidazol-6-on,

5-Äthyl-7-methyl-2-(3-pyridyl)-6,7-dihydro-3H,5H-pyrrolo[2,3-f]benzimidazol-6-on,

5-Äthyl-7-methyl-2-(2-pyridyl)-6,7-dihydro-3H,5H-pyrrolo[2,3-f]benzimidazol-6-on,

5-Äthyl-2-(4-methoxy-phenyl)-7-methyl-6,7-dihydro-3H,5H-pyrrolo[2,3-f]benzimidazol-6-on,

5-Äthyl-2-(4-hydroxy-phenyl)-7-methyl-6,7-dihydro-3H,5H-pyrrolo[2,3-f]benzimidazol-6-on,

5-(2-Methoxy-äthyl)-7-methyl-2-(4-pyridyl)-6,7-dihydro-3H,5H-pyrrolo[2,3-f]benzimidazol-6-on,

5-(2-Methoxy-äthyl)-7-methyl-2-(3-pyridyl)-6,7-dihydro-3H,5H-pyrrolo[2,3-f]benzimidazol-6-on,

5-(2-Methoxy-äthyl)-7-methyl-2-(2-pyridyl)-6,7-dihydro-3H,5H-pyrrolo[2,3-f]benzimidazol-6-on,

5-(2-Methoxy-äthyl)-2-(4-methoxy-phenyl)-7-methyl-6,7-dihydro-3H,5H-pyrrolo[2,3-f]benzimidazol-6-on,

5-(2-Methoxy-äthyl)-2-(4-hydroxy-phenyl)-7-methyl-6,7-dihydro-3H,5H-pyrrolo[2,3-f]benzimidazol-6-on,

5-(2-Hydroxy-äthyl)-7-methyl-2-(4-pyridyl)-6,7-dihydro-3H,5H-pyrrolo[2,3-f]benzimidazol-6-on,

5-(2-Hydroxy-äthyl)-7-methyl-2-(3-pyridyl)-6,7-dihydro-3H,5H-pyrrolo[2,3-f]benzimidazol-6-on,

5-(2-Hydroxy-äthyl)-7-methyl-2-(2-pyridyl)-6,7-dihydro-3H,5H-pyrrolo[2,3-f]benzimidazol-6-on,

5-(2-Hydroxy-äthyl)-2-(4-methoxy-phenyl)-7-methyl-6,7-dihydro-3H,5H-pyrrolo[2,3-f]benzimidazol-6-on,

5-(2-Hydroxy-äthyl)-2-(4-hydroxy-phenyl)-7-methyl-6,7-dihydro-3H,5H-pyrrolo[2,3-f]benzimidazol-6-on,

2-(2-Pyridyl)-5,7,7-trimethyl-6,7-dihydro-3H,5H-pyrrolo[2,3-f]benzimidazol-6-on,

5-Äthyl-7,7-dimethyl-2-(2-pyridyl)-6,7-dihydro-3H,5H-pyrrolo[2,3-f]-benzimidazol-6-on,

7,7-Dimethyl-2-(4-hydroxy-phenyl)-5-(2-methoxy-äthyl)-6,7-dihydro-3H,5H-pyrrolo[2,3-f]benzimidazol-6-on,

7,7-Dimethyl-5-(2-hydroxy-äthyl)-2-(4-pyridyl)-6,7-dihydro-3H,5H-pyrrolo[2,3-f]benzimidazol-6-on,

7,7-Dimethyl-5-(2-hydroxy-äthyl)-2-(3-pyridyl)-6,7-dihydro-3H,5H-pyrrolo[2,3-f]benzimidazol-6-on,

7,7-Dimethyl-5-(2-hydroxy-äthyl)-2-(2-pyridyl)-6,7-dihydro-3H,5H-pyrrolo[2,3-f]benzimidazol-6-on,

7,7-Dimethyl-5-(2-hydroxy-äthyl)-2-(4-methoxy-phenyl)-6,7-dihydro-3H,5H-pyrrolo[2,3-f]benzimidazol-6-on,

7,7-Dimethyl-5-(2-hydroxy-äthyl)-2-(4-hydroxy-phenyl)-6,7-dihydro-3H,5H-pyrrolo[2,3-f]benzimidazol-6-on,

1,5-Dimethyl-2-(4-pyridyl)-6,7-dihydro-1H,5H-pyrrolo[2,3-f]benzimidazol-6-on,

1,5-Dimethyl-2-(3-pyridyl)-6,7-dihydro-1H,5H-pyrrolo[2,3-f]benzimidazol-6-on,

1,5-Dimethyl-2-(2-pyridyl)-6,7-dihydro-1H,5H-pyrrolo[2,3-f]benzimidazol-6-on,

1,5-Dimethyl-2-(4-methoxy-phenyl)-6,7-dihydro-1H,5H-pyrrolo[2,3-f]benzimidazol-6-on,

1,5-Dimethyl-2-(4-hydroxy-phenyl)-6,7-dihydro-1H,5H-pyrrolo[2,3-f]benzimidazol-6-on,

5-Äthyl-1-methyl-2-(4-pyridyl)-6,7-dihydro-1H,5H-pyrrolo[2,3-f]benzimidazol-6-on,

5-Äthyl-1-methyl-2-(3-pyridyl)-6,7-dihydro-1H,5H-pyrrolo[2,3-f]benzimidazol-6-on,

5-Äthyl-1-methyl-2-(2-pyridyl)-6,7-dihydro-1H,5H-pyrrolo[2,3-f]benzimidazol-6-on,

5-Äthyl-2-(4-methoxy-phenyl)-1-methyl-6,7-dihydro-1H,5H-pyrrolo[2,3-f]benzimidazol-6-on,

5-Äthyl-2-(4-hydroxy-phenyl)-1-methyl-6,7-dihydro-1H,5H-pyrrolo[2,3-f]benzimidazol-6-on,

5-(2-Methoxy-äthyl)-1-methyl-2-(4-pyridyl)-6,7-dihydro-1H,5H-pyrrolo[2,3-f]benzimidazol-6-on,

5-(2-Methoxy-äthyl)-1-methyl-2-(3-pyridyl)-6,7-dihydro-1H,5H-pyrrolo[2,3-f]benzimidazol-6-on,

5-(2-Methoxy-äthyl)-1-methyl-2-(2-pyridyl)-6,7-dihydro-1H,5H-pyrrolo[2,3-f]benzimidazol-6-on,

5-(2-Methoxy-äthyl)-2-(4-methoxy-phenyl)-1-methyl-6,7-dihydro1H.5H-pyrrolo[2,3-f]benzimidazol-6-on,

5-(2-Methoxy-äthyl)-2-(4-hydroxy-phenyl)-1-methyl-6,7-dihydro-1H,5H-pyrrolo[2,3-f]benzimidazol-6-on,

5-(2-Hydroxy-äthyl)-1-methyl-2-(4-pyridyl)-6,7-dihydro-1H,5H-pyrrolo[2,3-f]benzimidazol-6-on,

5-(2-Hydroxy-äthyl)-1-methyl-2-(3-pyridyl)-6,7-dihydro-1H,5H-pyrrolo[2.3-f]benzimidazol-6-on,

5-(2-Hydroxy-äthyl)-1-methyl-2-(2-pyridyl)-6,7-dihydro-1H,5H-pyrrolo[2,3-f]benzimidazol-6-on,

5-(2-Hydroxy-äthyl)-2-(4-methoxy-phenyl)-1-methyl-6,7-dihydro-1H,5H-pyrrolo[2,3-f]benzimidazol-6-on,

5-(2-Hydroxy-äthyl)-2-(4-hydroxy-phenyl)-1-methyl-6,7-dihydro-1H,5H-pyrrolo[2,3-f]benzimidazol-6-on,

2-(4-Pyridyl)-1,5,7-trimethyl-6,7-dihydro-1H,5H-pyrrolo[2,3-f]benzimidazol-6-on,

2-(3-Pyridyl)-1,5,7-trimethyl-6,7-dihydro-1H,5H-pyrrolo[2,3-f]benzimidazol-6-on,

2-(2-Pyridyl)-1,5,7-trimethyl-6,7-dihydro-1H,5H-pyrrolo[2,3-f]benzimidazol-6-on,

2-(4-Methoxy-phenyl)-1,5,7-trimethyl-6,7-dihydro-1H,5H-pyrrolo[2,3-f]benzimidazol-6-on,

2-(4-Hydroxy-phenyl)-1,5,7-trimethyl-6,7-dihydro-1H,5H-pyrrolo[2,3-f]benzimidazol-6-on,

5-Äthyl-1,7-dimethyl-2-(4-pyridyl)-6,7-dihydro-1H,5H-pyrrolo[2,3-f]benzimidazol-6-on,

5-Äthyl-1,7-dimethyl-2-(3-pyridyl)-6,7-dihydro-1H,5H-pyrrolo[2,3-f]benzimidazol-6-on,

5-Äthyl-1,7-dimethyl-2-(2-pyridyl)-6,7-dihydro-1H,5H-pyrrolo[2,3-f]benzimidazol-6-on,

5-Äthyl-1,7-dimethyl-2-(4-methoxy-phenyl)-6,7-dihydro-1H,5H-pyrrolo[2,3-f]benzimidazol-6-on,

5-Äthyl-1,7-dimethyl-2-(4-hydroxy-phenyl)-6,7-dihydro-1H,5H-pyrrolo[2,3-f]benzimidazol-6-on,

1,7-Dimethyl-5-(2-methoxy-äthyl)-2-(4-pyridyl)-6,7-dihydro-1H,5H-pyrrolo[2,3-f]benzimidazol-6-on,

1,7-Dimethyl-5-(2-methoxy-äthyl)-2-(3-pyridyl)-6,7-dihydro-1H,5H-pyrrolo[2,3-f]benzimidazol-6-on,

1,7-Dimethyl-5-(2-methoxy-äthyl)-2-(2-pyridyl)-6,7-dihydro-1H,5H-pyrrolo[2,3-f]benzimidazol-6-on,

1,7-Dimethyl-5-(2-methoxy-äthyl)-2-(4-methoxy-phenyl)-6,7-dihydro-1H,5H-pyrrolo[2,3-f]benzimidazol-6-on,

1,7-Dimethyl-2-(4-hydroxy-phenyl)-5-(2-methoxy-äthyl)-6,7-dihydro-1H,5H-pyrrolo[2,3-f]benzimidazol-6-on,

1,7-Dimethyl-5-(2-hydroxy-äthyl)-2-(4-pyridyl)-6,7-dihydro-1H,5H-pyrrolo[2,3-f]benzimidazol-6-on,

1,7-Dimethyl-5-(2-hydroxy-äthyl)-2-(3-pyridyl)-6,7-dihydro-1H,5H-pyrrolo[2,3-f]benzimidazol-6-on,

1,7-Dimethyl-5-(2-hydroxy-äthyl)-2-(2-pyridyl)-6,7-dihydro-1H,5H-pyrrolo[2,3-f]benzimidazol-6-on,

1,7-Dimethyl-5-(2-hydroxy-äthyl)-2-(4-methoxy-phenyl)-6,7-dihydro-1H,5H-pyrrolo[2,3-f]benzimidazol-6-on,

1,7-Dimethyl-5-(2-hydroxy-äthyl)-2-(4-hydroxy-phenyl)-6,7-dihydro-1H,5H-pyrrolo[2,3-f]benzimidazol-6-on,

2-(3-Pyridyl)-1,5,7,7-tetramethyl-6,7-dihydro-1H,5H-pyrrolo[2,3-f]benzimidazol-6-on,

2-(2-Pyridyl)-1,5,7,7-tetramethyl-6,7-dihydro-1H,5H-pyrrolo[2,3-f]benzimidazol-6-on,

2-(4-Methoxy-phenyl)-1,5,7,7-tetramethyl-6,7-dihydro-1H,5H-pyrrolo[2,3-f]benzimidazol-6-on,

2-(4-Hydroxy-phenyl)-1,5,7,7-tetramethyl-6,7-dihydro-1H,5H-pyrrolo[2,3-f]benzimidazol-6-on,

5-Äthyl-2-(4-pyridyl)-1,7,7-trimethyl-6,7-dihydro-1H,5H-pyrrolo[2,3-f]benzimidazol-6-on,

5-Äthyl-2-(3-pyridyl)-1,7,7-trimethyl-6,7-dihydro-1H,5H-pyrrolo[2,3-f]benzimidazol-6-on,

5-Äthyl-2-(2-pyridyl)-1,7,7-trimethyl-6,7-dihydro-1H,5H-pyrrolo[2,3-f]benzimidazol-6-on,

5-Äthyl-2-(4-methoxy-phenyl)-1,7,7-trimethyl-6,7-dihydro-1H,5H-pyrrolo[2,3-f]benzimidazol-6-on,

5-Äthyl--(4-hydroxy-phenyl)-1,7,7-trimethyl-6,7-dihydro-1H,5H-pyrrolo[2,3-f]benzimidazol-6-on,

5-(2-Methoxy-äthyl)-2-(4-pyridyl)-1,7,7-trimethyl-6,7-dihydro-1H,5H-pyrrolo[2,3-f]benzimidazol-6-on,

5-(2-Methoxy-äthyl)-2-(3-pyridyl)-1,7,7-trimethyl-6,7-dihydro-1H,5H-pyrrolo[2,3-f]benzimidazol-6-on,

5-(2-Methoxy-äthyl)-2-(2-pyridyl)-1,7,7-trimethyl-6,7-dihydro-1H,5H-pyrrolo[2,3-f]benzimidazol-6-on,

5-(2-Methoxy-äthyl)-2-(4-methoxy-phenyl)-1,7,7-trimethyl-6,7-dihydro-1H,5H-pyrrolo[2,3-f]benzimidazol-6-on,

5-(2-Methoxy-äthyl)-2-(4-hydroxy-phenyl)-1,7,7-trimethyl-6,7-dihydro-1H,5H-pyrrolo[2,3-f]benzimidazol-6-on,

5-(2-Hydroxy-äthyl)-2-(4-pyridyl)-1,7,7-trimethyl-6,7-dihydro-1H,5H-pyrrolo[2,3-f]benzimidazol-6-on,

5-(2-Hydroxy-äthyl)-2-(3-pyridyl)-1,7,7-trimethyl-6,7-dihydro-1H,5H-pyrrolo[2,3-f]benzimidazol-6-on,

5-(2-Hydroxy-äthyl)-2-(2-pyridyl)-1,7,7-trimethyl-6,7-dihydro-1H,5H-pyrrolo[2,3-f]benzimidazol-6-on,

5-(2-Hydroxy-äthyl)-2-(4-methoxy-phenyl)-1,7,7-trimethyl-6,7-dihydro-1H,5H-pyrrolo[2,3-f]benzimidazol-6-on,

5-(2-Hydroxy-äthyl)-2-(4-hydroxy-phenyl)-1,7,7-trimethyl-6,7-dihydro-1H,5H-pyrrolo[2,3-f]benzimidazol-6-on,

3,5-Dimethyl-2-(4-pyridyl)-6,7-dihydro-3H,5H-pyrrolo[2,3-f]benzimidazol-6-on,

3,5-Dimethyl-2-(3-pyridyl)-6,7-dihydro-3H,5H-pyrrolo[2,3-f]benzimidazol-6-on,

3,5-Dimethyl-2-(2-pyridyl)-6,7-dihydro-3H,5H-pyrrolo[2,3-f]benzimidazol-6-on,

3,5-Dimethyl-2-(4-methoxy-phenyl)-6,7-dihydro-3H,5H-pyrrolo[2,3-f]benzimidazol-6-on,

3,5-Dimethyl-2-(4-hydroxy-phenyl)-6,7-dihydro-3H,5H-pyrrolo[2,3-f]benzimidazol-6-on,

5-Äthyl-3-methyl-2-(4-pyridyl)-6,7-dihydro-3H,5H-pyrrolo[2,3-f]benzimidazol-6-on,

5-Äthyl-3-methyl-2-(3-pyridyl)-6,7-dihydro-3H,5H-pyrrolo[2,3-f]benzimidazol-6-on,

5-Äthyl-3-methyl-2-(2-pyridyl)-6,7-dihydro-3H,5H-pyrrolo[2,3-f]benzimidazol-6-on,

5-Äthyl-2-(4-methoxy-phenyl)-3-methyl-6,7-dihydro-3H,5H-pyrrolo[2,3-f]benzimidazol-6-on,

5-Äthyl-2-(4-hydroxy-phenyl)-3-methyl-6,7-dihydro-3H,5H-pyrrolo[2,3-f]benzimidazol-6-on,

5-(2-Methoxy-äthyl)-3-methyl-2-(4-pyridyl)-6,7-dihydro-3H.5H-pyrrolo[2,3-f]benzimidazol-6-on,

5-(2-Methoxy-äthyl)-3-methyl-2-(3-pyridyl)-6,7-dihydro-3H,5H-pyrrolo[2,3-f]benzimidazol-6-on,

5-(2-Methoxy-äthyl)-3-methyl-2-(2-pyridyl)-6,7-dihydro-3H,5H-pyrrolo[2,3-f]benzimidazol-6-on,

5-(2-Methoxy-äthyl)-2-(4-methoxy-phenyl)-3-methyl-6,7-dihydro3H,5H-pyrrolo[2,3-f]benzimidazol-6-on,

2-(4-Hydroxy-phenyl)-5-(2-methoxy-äthyl)-3-methyl-6,7-dihydro3H.5H-pyrrolo[2,3-f]benzimidazol-6-on,

5-(2-Hydroxy-äthyl)-3-methyl-2-(4-pyridyl)-6,7-dihydro-3H.5H-pyrrolo[2,3-f]benzimidazol-6-on,

5-(2-Hydroxy-äthyl)-3-methyl-2-(3-pyridyl)-6,7-dihydro-3H,5H-pyrrolo[2,3-f]benzimidazol-6-on,

5-(2-Hydroxy-äthyl)-3-methyl-2-(2-pyridyl)-6,7-dihydro-3H,5H-pyrrolo[2,3-f]benzimidazol-6-on,

5-(2-Hydroxy-äthyl)-2-(4-methoxy-phenyl)-3-methyl-6,7-dihydro-3H,5H-pyrrolo[2,3-f]benzimidazol-6-on,

5-(2-Hydroxy-äthyl)-2-(4-hydroxy-phenyl)-3-methyl-6,7-dihydro-3H,5H-pyrrolo[2,3-f]benzimidazol-6-on,

2-(4-Pyridyl)-3,5,7-trimethyl-6,7-dihydro-3H,5H-pyrrolo[2,3-f)benzimidazol-6-on,

2-(3-Pyridyl)-3,5,7-trimethyl-6,7-dihydro-3H,5H-pyrrolo[2,3-f]benzimidazol-6-on,

2-(2-Pyridyl)-3,5,7-trimethyl-6,7-dihydro-3H,5H-pyrrolo[2,3-f]benzimidazol-6-on,

2-(4-Methoxy-phenyl)-3,5,7-trimethyl-6,7-dihydro-3H,5H-pyrrolo[2,3-f]benzimidazol-6-on,

2-(4-Hydroxy-phenyl)-3,5,7-trimethyl-6,7-dihydro-3H,5H-pyrrolo[2,3-f]benzimidazol-6-on,

5-Äthyl-3,7-dimethyl-2-(4-pyridyl)-6,7-dihydro-3H,5H-pyrrolo[2,3-f]benzimidazol-6-on,

5-Äthyl-3,7-dimethyl-2-(3-pyridyl)-6,7-dihydro-3H,5H-pyrrolo[2,3-f]benzimidazol-6-on,

5-Äthyl-3,7-dimethyl-2-(2-pyridyl)-6,7-dihydro-3H,5H-pyrrolo[2,3-f]benzimidazol-6-on,

5-Äthyl-3,7-dimethyl-2-(4-methoxy-phenyl)-6,7-dihydro-3H,5H-pyrrolo[2,3-f]benzimidazol-6-on,

5-Äthyl-3,7-dimethyl-2-(4-hydroxy-phenyl)-6,7-dihydro-3H,5H-pyrrolo[2,3-f]benzimidazol-6-on,

3,7-Dimethyl-5-(2-methoxy-äthyl)-2-(4-pyridyl)-6,7-dihydro-3H,5H-pyrrolo[2,3-f]benzimidazol-6-on,

3,7-Dimethyl-5-(2-methoxy-äthyl)-2-(3-pyridyl)-6,7-dihydro-3H,5H-pyrrolo[2,3-f]benzimidazol-6-on,

3,7-Dimethyl-5-(2-methoxy-äthyl)-2-(2-pyridyl)-6,7-dihydro-3H,5H-pyrrolo[2,3-f]benzimidazol-6-on,

3,7-Dimethyl-5-(2-methoxy-äthyl)-2-(4-methoxy-phenyl)-6,7-di-hydro-3H,5H-pyrrolo[2,3-f]benzimidazol-6-on,

3,7-Dimethyl-2-(4-hydroxy-phenyl)-5-(2-methoxy-äthyl)-6,7-dihydro-3H,5H-pyrrolo[2,3-f]benzimidazol-6-on,

3,7-Dimethyl-5-(2-hydroxy-äthyl)-2-(4-pyridyl)-6,7-dihydro-3H.5H-pyrrolo[2,3-f]benzimidazol-6-on,

3,7-Dimethyl-5-(2-hydroxy-äthyl)-2-(3-pyridyl)-6,7-dihydro-3H,5H-pyrrolo[2,3-f]benzimidazol-6-on,

3,7-Dimethyl-5-(2-hydroxy-äthyl)-2-(2-pyridyl)-6,7-dihydro-3H,5H-pyrrolo[2,3-f]benzimidazol-6-on,

3,7-Dimethyl-5-(2-hydroxy-äthyl)-2-(4-methoxy-phenyl)-6,7-dihydro-3H,5H-pyrrolo[2,3-f]benzimidazol-6-on,

3,7-Dimethyl-5-(2-hydroxy-äthyl)-2-(4-hydroxy-phenyl)-6,7-dihydro-3H,5H-pyrrolo[2,3-f]benzimidazol-6-on,

2-(3-Pyridyl)-3,5,7,7-tetramethyl-6,7-dihydro-3H,5H-pyrrolo[2,3-f]benzimidazol-6-on,

2-(2-Pyridyl)-3,5,7,7-tetramethyl-6,7-dihydro-3H,5H-pyrrolo[2,3-f]benzimidazol-6-on,

2-(4-Methoxy-phenyl)-3,5,7,7-tetramethyl-6,7-dihydro-3H,5H-pyrrolo[2,3-f]benzimidazol-6-on,

2-(4-Hydroxy-phenyl)-3,5,7,7-tetramethyl-6,7-dihydro-3H,5H-pyrrolo[2,3-f]benzimidazol-6-on,

5-Äthyl-2-(4-pyridyl)-3,7,7-trimethyl-6,7-dihydro-3H,5H-pyrrolo[2,3-f]benzimidazol-6-on,

5-Äthyl-2-(3-pyridyl)-3,7,7-trimethyl-6,7-dihydro-3H,5H-pyrrolo[2,3-f]benzimidazol-6-on,

5-Äthyl-2-(2-pyridyl)-3,7,7-trimethyl-6,7-dihydro-3H,5H-pyrrolo[2,3-f]benzimidazol-6-on,

5-Äthyl-2-(4-methoxy-phenyl)-3,7,7-trimethyl-6,7-dihydro-3H,5H-pyrrolo[2,3-f]benzimidazol-6-on,

5-Äthyl-2-(4-hydroxy-phenyl)-3,7,7-trimethyl-6,7-dihydro-3H,5H-pyrrolo[2,3-f]benzimidazol-6-on,

5-(2-Methoxy-äthyl)-2-(4-pyridyl)-3,7,7-trimethyl-6,7-dihydro-3H,5H-pyrrolo[2,3-f]benzimidazol-6-on,

5-(2-Methoxy-äthyl)-2-(3-pyridyl)-3,7,7-trimethyl-6,7-dihydro-3H,5H-pyrrolo[2,3-f]benzimidazol-6-on,

5-(2-Methoxy-äthyl)-2-(2-pyridyl)-3,7,7-trimethyl-6,7-dihydro-3H,5H-pyrrolo[2,3-f]benzimidazol-6-on,

5-(2-Methoxy-äthyl)-2-(4-methoxy-phenyl)-3,7,7-trimethyl-6,7-dihydro-3H,5H-pyrrolo[2,3-f]benzimidazol-6-on,

2-(4-Hydroxy-phenyl)-5-(2-methoxy-äthyl)-3,7,7-trimethyl-6,7-dihydro-3H,5H-pyrrolo[2,3-f]benzimidazol-6-on,

5-(2-Hydroxy-äthyl)-2-(4-pyridyl)-3,7,7-trimethyl-6,7-dihydro-3H,5H-pyrrolo[2,3-f]benzimidazol-6-on,

5-(2-Hydroxy-äthyl)-2-(3-pyridyl)-3,7,7-trimethyl-6,7-dihydro-3H,5H-pyrrolo[2,3-f]benzimidazol-6-on,

5-(2-Hydroxy-äthyl)-2-(2-pyridyl)-3,7,7-trimethyl-6,7-dihydro-3H,5H-pyrrolo[2,3-f]benzimidazol-6-on,

5-(2-Hydroxy-äthyl)-2-(4-methoxy-phenyl)-3,7,7-trimethyl-6,7-dihydro-3H,5H-pyrrolo[2,3-f]benzimidazol-6-on,

5-(2-Hydroxy-äthyl)-2-(4-hydroxy-phenyl)-3,7,7-trimethyl-6,7-dihydro-3H,5H-pyrrolo[2,3-f]benzimidazol-6-on,

5-Benzyl-7,7-dimethyl-2-(3-pyridyl)-6,7-dihydro-3H,5H-pyrrolo[2,3-f]benzimidazol-6-on,

5-Benzyl-7,7-dimethyl-2-(2-pyridyl)-6,7-dihydro-3H,5H-pyrrolo[2,3-f]benzimidazol-6-on und

5-Benzyl-7,7-dimethyl-2-(4-hydroxy-phenyl)-6,7-dihydro-3H,5H-pyrrolo[2,3-f]benzimidazol-6-on, deren 3H-Tautomere und deren Säureadditionssalze.

Bevorzugte Verbindungen der obigen Formel I sind jedoch diejenigen, in denen $R_1$ ein Wasserstoffatom darstellt, insbesondere jedoch diejenigen Verbindungen der Formel I, in denen

$R_1$ ein Wasserstoffatom,

$R_2$ ein Wasserstoffatom, eine Alkylgruppe mit 1 bis 3 Kohlenstoffatomen, eine durch eine Phenyl-, Pyridyl- oder Thienylgruppe substituierte Alkylgruppe mit 1 oder 2 Kohlenstoffatomen im Alkylteil, eine endständig durch eine Phenyl-oder Pyridylgruppe substituierte Vinylengruppe, eine gegebenenfalls durch eine Methyl-,

Amino-, Methylamino-, Dimethylamino-oder Morpholinogruppe substituierte Pyridylgruppe, eine durch 2 Halogenatome oder durch ein Halogenatom und eine Morpholinogruppe substituierte Pyridylgruppe, eine gegebenenfalls durch eine Methylgruppe substituierte Furyl-, Thienyl-, Pyrrolyl-, Oxazolyl-, Pyrimidinyl-, Pyrazinyl-, Pyridazinyl-, Chinolyl-oder Acridinylgruppe, eine durch eine Phenylgruppe oder durch ein Halogenatom und eine Methoxy-oder Trifluormethylgruppe substituierte Chinolylgruppe, eine gegebenenfalls am N-Atom durch eine Acetylgruppe substituierte Piperidinyl-oder 1,2,3,4-Tetrahydro-chinolylgruppe oder eine 5,6,7,8-Tetrahydro-chinolylgruppe, eine gegebenenfalls durch ein Halogenatom, eine Hydroxy-, Methoxy-, Methylmercapto-, Methylsulfinyl-, Methylsulfonyl-, Nitro-, Amino-, Methylamino-, Dimethylamino-, Acetylamino-oder Cyanogruppe substituierte Phenylgruppe, eine durch ein Halogenatom, eine Methoxy-, Methylmercapto-, Methylsulfinyloder Methylsulfonylgruppe disubstituierte Phenylgruppe, wobei die Substituenten gleich oder verschieden sein können, eine Hydroxy-di-tert.Butylphenyl-oder Amino-dihalogenphenylgruppe,

$R_3$ eine Alkylgruppe mit 1 bis 5 Kohlenstoffatomen, eine Benzyl-, 2-Hydroxy-äthyl-oder 2-Methoxy-äthylgruppe,

$R_4$ und $R_5$, die gleich oder verschieden sein können, Wasserstoffatome oder Methylgruppen bedeuten, deren 3H-Tautomere und deren Säureadditionssalze.

Besonders bevorzugte Verbindungen der Formel I sind jedoch diejenigen, in denen

$R_1$ ein Wasserstoffatom,

$R_2$ eine durch eine Cyano-, Dimethylamino-, Methoxy-, Methylmercapto-, Methylsulfinyl-oder Methylsulfonylgruppe substituierte Phenylgruppe, eine gegebenenfalls durch eine Methyl-, Amino-, Methylamino-, Dimethylamino-oder Morpholinogruppe substituierte Pyridylgruppe, eine durch 2 Halogenatome oder durch ein Halogenatom und eine Morpholinogruppe substituierte Pyridylgruppe, eine gegebenenfalls durch eine Methylgruppe substituierte Furyl-, Thienyl-, Pyrrolyl-, Oxazolyl-, Pyrimidinyl-, Pyrazinyl-, Pyridazinyl-, Chinolyl-oder Acridinylgruppe,

$R_3$ eine Alkylgruppe mit 1 bis 5 Kohlenstoffatomen,

$R_4$ ein Wasserstoffatom oder eine Methylgruppe und

$R_5$ eine Methylgruppe bedeuten, insbesondere jedoch diejenigen, in denen

$R_1$ ein Wasserstoffatom,

$R_2$ eine in 4-Stellung durch eine Cyano-, Dimethylamino-, Methylsulfinyl-oder Methylsulfonylgruppe substituierte Phenylgruppe, eine Furyl-2-, Thienyl-2-, Pyridyl-, 4-Pyrimidinyl-oder 4-Methyl-oxazol-5-yl-gruppe,

$R_3$ eine Alkylgruppe mit 1 bis 3 Kohlenstoffatomen,

$R_4$ und $R_5$ jeweils eine Methylgruppe bedeuten, deren 3H-Tautomere und deren Säureadditionssalze.

Erfindungsgemäß erhält man die neuen Verbindungen nach folgenden Verfahren:

a) Cyclisierung einer Verbindung der Formel

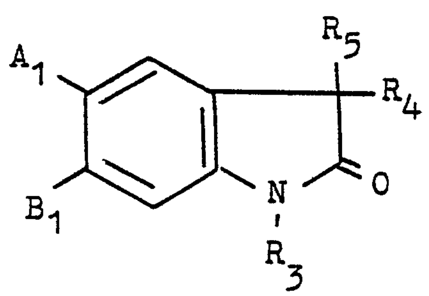

(II)

in der

$R_3$ bis $R_5$ wie eingangs deginiert sind,

einer der Reste $A_1$ oder $B_1$ eine $R_1NH$-Gruppe, wobei
$R_1$ wie eingangs deginiert ist, und
der andere der Reste $A_1$ oder $B_1$ eine

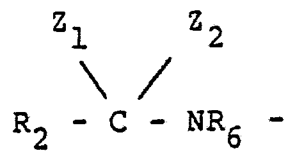

Gruppe bedeuten, wobei
$R_2$ wie eingangs deginiert ist und
$R_6$ ein Wasserstoffatom oder auch, falls $R_1$ ein Wasserstoffatom darstellt, eine gegebenenfalls durch eine Phenylgruppe substituierte Alkylgruppe mit 1 bis 3 Kohlenstoffatomen,

$Z_1$ und $Z_2$, die gleich oder verschieden sein können, nukleophile Austrittsgruppen wie gegebenenfalls substituierte Amino-, Alkoxy-, Phenylalkoxy-, Phenoxy-, Alkylmercapto-, Phenylalkylmercapto-oder Phenylthiogruppen oder

$Z_1$ und $Z_2$ zusammen ein Sauerstoff-oder Schwefelatom, eine gegebenenfalls durch eine Alkyl-oder Phenylalkylgruppe substituierte Iminogruppe, wobei die vorstehend erwähnten Alkylteile jeweils 1 bis 3 Kohlenstoffatome enthalten können, oder eine Alkylendioxigruppe mit 2 oder 3 Kohlenstoffatomen bedeuten.

Für $Z_1$ und $Z_2$ kommt beispielsweise jeweils die der Methoxy-, Ethoxy-, Propoxy-, Benzyloxy-, Methylmercapto-, Ethylmercapto-, Propylmercapto-, Phenylmercapto-oder Benzylmercaptogruppe oder

für $Z_1$ und $Z_2$ zusammen mit dem Kohlenstoffatom beispielsweise eine Carbonyl-oder Thiocarbonylgruppe in Betracht.

Die Umsetzung wird zweckmäßigerweise in einem Lösungsmittel oder Lösungsmittelgemisch wie Ethanol, Isopropanol, Eisessig, Tetrahydrofuran, Dioxan, Benzol, Toluol, Xylol, Chlorbenzol, Glycol, Glycolmonomethyläther, Glycoldimethyläther, Diäthylenglycoldimethylether, Dimethylformamid, Tetralin oder Sulfolan gegebenenfalls in Gegenwart eines Kondensationsmittels wie Phosphoroxychlorid, Thionylchlorid, Sulfurylchlorid, Salzsäure, Schwefelsäure, Phosphorsäure, Polyphosphorsäure, p-Toluolsulfonsäure, Eisessig, Essigsäureanhydrid, N,N'-Dicyclohexyl-carbodiimid, Carbonyldiimidazol, Kaliummethylat oder Kalium-tert.butylat bei Temperaturen zwischen 0 und 300°C, vorzugsweise jedoch bei der Siedetemperatur des Reaktionsgemisches, z.B. bei Temperaturen zwischen 50 und 285°C, durchgeführt. Die Umsetzung kann jedoch auch in der Schmelze durchgeführt werden.

Besonders vorteilhaft wird die Umsetzung jedoch in der Weise durchgeführt, daß eine Ausgangsverbindung der Formel II im Reaktionsgemisch entweder durch Reduktion einer entsprechenden Nitroverbindung, z. B. durch Reduktion mit Wasserstoff in Gegenwart eines Hydrierungskatalysators

oder durch Umsetzung einer entsprechenden Diaminoverbindung mit einem Überschuß des eingesetzten Acylierungsmittels, z.B. mit einem Überschuß des entsprechenden Nitrils, Anhydrids, Esters, Thioesters, Orthoesters, Amids, Thioamids, Halogenids oder Methojodids, hergestellt wird.

Hierbei wird die Reduktion einer Nitrogruppe vorzugsweise in einem geeigneten Lösungsmittel wie Eisessig, Wasser, Äthanol oder Wasser/Eisessig zweckmäßigerweise mit nascierendem Wasserstoff, z.B. in Gegenwart von Zink/Eisessig, Zinn/Salzsäure oder Zinn(II)chlorid/Salzsäure, oder mit katalytisch angeregtem Wasserstoff, z.B. mit Wasserstoff in Gegenwart von Platin, Palladium oder Raney-Nickel, bei Temperaturen zwischen 0 und 150°C, vorzugsweise bei Temperaturen zwischen 20 und 125°C, bzw.

die Acylierung zweckmäßigerweise in einem Lösungsmittel wie Methylenchlorid, Chloroform, Tetrachlorkohlenstoff, Äther, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureäthylester, Thionylchlorid, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie

Natriumcarbonat oder einer tertiären organischen Base wie Triäthylamin oder Pyridin, welche gleichzeitig als Lösungsmittel dienen können, bei Temperaturen zwischen -25 und 250°C, vorzugsweise jedoch bei Temperaturen zwischen -10°C und der Siedetemperatur des verwendeten Lösungsmittels, durchgeführt. Die Umsetzung kann auch ohne Lösungsmittel durchgeführt werden, desweiteren kann während der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.

Bei der Reduktion können im Rest $R_2$ vorhandene Doppelbindungen mitreduziert werden bzw. bei der Acylierung in Gegenwart eines halogenhaltigen Kondensationsmittels wie Phosphoroxychlorid vorhandene Hydroxygruppen in Halogenatome übergeführt werden.

b) Umsetzung einer Verbindung der Formel

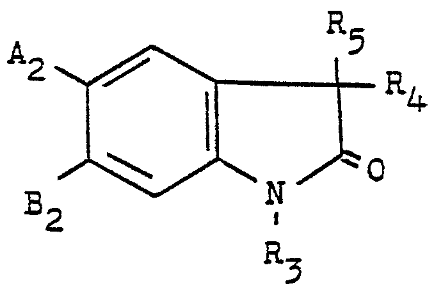

(III)

in der

$R_3$, $R_4$ und $R_5$ wie eingangs deginiert sind,
einer der Reste $A_2$ oder $B_2$ eine $R_1NH$-Gruppe, wobei
$R_1$ wie eingangs definiert ist, und
der andere der Reste $A_2$ oder $B_2$ eine $NH_2$-Gruppe bedeuten, mit einem Aldehyd der Formel

$R_2$-CHO ,(IV)

in der
$R_2$ wie eingangs definiert ist, und anschließende Oxidation.

Die Umsetzung wird zweckmäßigerweise in einem geeigneten Lösungsmittel wie Methanol, Ethanol, Eisessig oder Ethylenchlorid bei Temperaturen zwischen 20 und 100°C durchgeführt. Die anschließende Oxidation wird mit einem Oxidationsmittel wie Sauerstoff, Wasserstoffperoxid oder einer Persäure wie m-Chlor-perbenzoesäure bei Temperaturen zwischen 20 und 100°C, vorzugsweise bei Temperaturen zwischen 40 und 80°C, durchgeführt.

Erhält man erfindungsgemäß eine Verbindung der Formel I, in der $R_1$ ein Wasserstoffatom darstellt und/oder $R_2$ eine Hydroxy-oder Mercaptogruppe enthält, so kann diese mittels Alkylierung in die entsprechende Alkylverbindung übergeführt werden und/oder

eine Verbindung der Formel I, in der $R_2$ eine Aminogruppe enthält, so kann diese mittels Alkanoylierung in die entsprechende Alkanoylverbindung übergeführt werden und/oder

eine Verbindung der Formel I, in der $R_2$ eine endständig durch eine Phenyl-oder Pyridylgruppe substituierte Vinylengruppe darstellt, so kann diese mittels katalytischer Hydrierung in eine entsprechende Verbindung der Formel I, in der $R_2$ eine endständig durch eine Phenyl-oder Pyridylgruppe substituierte Ethylengruppe darstellt, übergeführt werden und/oder

eine Verbindung der Formel I, in der $R_1$ eine Benzylgruppe darstellt, so kann diese mittels katalytischer Hydrierung in eine entsprechende Verbindung der Formel I, in der $R_1$ ein Wasserstoff darstellt, übergeführt werden und/oder

eine Verbindung der Formel I, in der $R_2$ ein Pyridinring darstellt, so kann dieser mittels katalytischer Hydrierung in eine entsprechende Verbindung der Formel I, in der $R_2$ eine Piperidinylgruppe darstellt, übergeführt werden und/oder

eine Verbindung der Formel I, in der $R_2$ eine Alkoxyphenyl-oder Dialkoxyphenylgruppe darstellt, so kann

diese mittels Etherspaltung in eine entsprechende Verbindung der Formel I, in der $R_2$ eine Hydroxyphenyl- oder Dihydroxyphenylgruppe darstellt, übergeführt werden und/oder

eine Verbindung der Formel I, in der $R_2$ eine Alkylmercaptophenyl-oder Dialkylmercaptophenylgruppe darstellt, so kann diese mittels Oxidation in eine entsprechende Verbin dung der Formel I, in der $R_2$ eine Alkylsulfinyl-, Alkylsulfonyl-, Dialkylsulfinyl-oder Dialkylsulfonyl-phenylgruppe darstellt, übergeführt werden.

Die nachträgliche Alkylierung wird in einem geeigneten Lösungsmittel wie Methanol, Ethanol, Diethylether, Aceton, Methylenchlorid, Tetrahydrofuran, Dioxan, Dimethylformamid oder Dimethylsulfoxid, gegebenenfalls in Gegenwart einer Base wie Natriumcarbonat, Kalium-tert.butylat, Triethylamin oder Pyridin, wobei die beiden letzteren gleichzeitig auch als Lösungsmittel dienen können, mit einem geeigneten Alkylierungsmittel wie Methyljodid, Dimethylsulfat, Ethylbromid, Diethylsulfat, n-Propylbromid, Isopropylbromid, Ethylenoxid, 2-Hydroxy-ethylbromid oder Formaldehyd/Ameisensäure oder in Gegenwart von Natriumcyanborhyrid, falls eine entsprechende Carbonylverbindung eingesetzt wird, bei Temperaturen zwischen 0 und 100°C, durchgeführt.

Die nachträgliche Alkanoylierung wird zweckmäßigerweise in einem Lösungsmittel wie Tetrahydrofuran, Dioxan, Benzol oder Dimethylformamid gegebenenfalls in Gegenwart eines Kondensationsmittels wie Thionylchlorid, N,N'-Dicyclohexyl-carbodiimid-oder Carbonyldiimidazol, vorzugsweise jedoch mit dem entsprechenden Säureanhydrid oder Säurehalogenid, bei Temperaturen zwischen 0 und 100°C, vorzugsweise jedoch bei Temperaturen zwischen 20 und 50°C, durchgeführt.

Die nachträgliche katalytische Hydrierung wird vorzugsweise mit Wasserstoff in Gegenwart eines Hydrierungskatalysators wie Platin, Palladium/Kohle oder Raney-Nickel in einem geeigneten Lösungsmittel, z.B. in Methanol, Ethanol, Tetrahydrofuran, Dioxan, Eisessig, Dimethylformamid oder Essigsäureethylester, bei Temperaturen zwischen 20 und 100°C, vorzugsweise bei 20 bis 50°C, durchgeführt.

Die nachträgliche Etherspaltung wird in Gegenwart einer Säure wie Salzsäure, Schwefelsäure, Pyridinhydrochlorid, Bortribromid, Aluminiumchlorid oder Zinn(IV)chlorid gegebenenfalls in einem geeigneten Lösungsmittel wie Methylenchlorid, Ethylenchlorid oder in einem Überschuß der eingesetzten Säure bei Temperaturen zwischen -10°C und der Siedetemperatur des Reaktionsgemisches durchgeführt. Bei der Ätherspaltung mit Pyridin-hydrochlorid wird eine Verbindung der Formel I, in der $R_1$ eine der eingangs erwähnten Alkoxyalkylgruppen darstellt, gleichzeitig in die entsprechende Pyridiniumverbindung übergeführt.

Die nachträgliche Oxidation wird vorzugsweise in einem Lösungsmittel oder Lösungsmittelgemisch, z.B. in Wasser, Wasser/Pyridin, Methanol, Ethanol, Aceton, Ameisensäure, Eisessig, verdünnter Schwefelsäure oder Trifluoressigsäure, je nach dem verwendeten Oxidationsmittel zweckmäßigerweise bei Temperaturen zwischen -80 und 100°C, durchgeführt.

Zur Herstellung einer Alkylsulfinylverbindung der Formel I wird die Oxidation zweckmäßigerweise mit einem Äquivalent des verwendeten Oxidationsmittels durchgeführt, z.8. mit Wasserstoffperoxid in Eisessig oder Ameisensäure bei 0 bis 20°C oder in Aceton bei 0 bis 60°C, mit einer Persäure wie Perameisensäure oder m-Chlor-perbenzoesäure in Eisessig oder Trifluoressigsäure bei 0 bis 20°C, mit Natriummetaperjodat in wässrigem Methanol oder Ethanol bei 15 bis 25°C oder mit tert.Butyl-hypochlorit in Methanol bei -80 bis -30°C.

Zur Herstellung einer Alkylsulfonylverbindung der Formel I wird die Oxidation zweckmäßigerweise mit einem bzw. mit mehr Äquivalenten des verwendeten Oxidationsmittels durchgeführt, z.B. mit Wasserstoff in Eisessig oder in Ameisensäure bei 20 bis 100°C oder in Aceton bei 0 bis 60°C, mit einer Persäure wie Perameisensäure oder m-Chlor-perbenzoesäure in Eisessig, Trifluoressigsäure oder Chloroform bei 0 bis 50°C, oder mit Bromsäure oder Kaliumpermanganat in Eisessig, Wasser/Schwefelsäure oder in Aceton bei 0 bis 20°C. Enthält somit eine eingesetzte Verbindung der Formel I eine Alkylmercaptogruppe, so wird die nachträgliche Oxidation vorzugsweise mit zwei oder mehr Äquivalenten des betreffenden Oxidationsmittels und ganz entsprechend mit mindestens einem Äquivalent durchgeführt, falls eine eingesetzte Verbindung der Formel I eine Alkylsulfinylgruppe enthält.

Desweiteren können die erhaltenen Verbindungen der Formel I in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Die als Ausgangsstoffe verwendeten Verbindungen der allgemeinen Formeln II bis IV erhält man nach literaturbekannten Verfahren.

So erhält man beispielsweise 5-Isonicotinoylamino-6-nitro1,3,3-trimethyl-indolin-2-on durch Umsetzung von 5-Acetamino-3,3-dimethyl-indolin-2-on mit Natriumhydrid oder Kalium-tert.butylat/Methyljodid in Dimethylformamid. Durch anschließende Nitrierung des so erhaltenen 5-Acetamino-1,3,3-trimethyl-indolin-2-on mit konz. Salpetersäure (d = 1,51) und nachfolgender Verseifung mit halbkonzentrierter Salzsäure erhält man 5-Amino-6-nitro-1,3,3-trimethyl-indolin-2-on, welches durch Reduktion in die entsprechende 5,6-Diaminoverbindung oder durch Reaktion mit Isonicotinsäurechlorid in 5-Isonicotinoylamino-6-nitro-1,3,3-trimethyl-indolin-2-on übergeführt wird (siehe Beispiele A bis D).

Die neuen Verbindungen der Formel I, deren optisch aktiven Antipoden und deren Säureadditionssalze, insbesondere deren physiologisch verträgliche Säureadditionssalze mit anorganischen oder organischen Säuren weisen, wie bereits eingangs erwähnt, wertvolle pharmakologische Eigenschaften auf, insbesondere bei einer leichten Beeinflußung des Blutdruckes eine positiv inotrope (cardiotonische) Wirkung und eine antithrombotische Wirkung.

Beispielsweise wurden die Verbindungen

A = 5-Äthyl-7,7-dimethyl-2-(4-pyridyl)-6,7-dihyro-3H,5H-pyrrolo[2,3-f]benzimidazol-6-on,
B = 5-Äthyl-7,7-dimethyl-2-(2-furyl)-6,7-dihydro-3H,5H-pyrrolo[2,3-f]benzimidazol-6-on,
C = 5-Äthyl-7,7-dimethyl-2-(4-methylsulfonyl-phenyl)-6,7-dihydro-3H,5H-pyrrolo[2,3-f]benzimidazol-6-on und
D = 5-Äthyl-7,7-dimethyl-2-(4-methyl-oxazol-5-yl)-6,7-dihydro-3H,5H-pyrrolo[2,3-f]benzimidazol-6-on

auf ihre biologischen Eigenschaften wie folgt untersucht:

1. Bestimmung der blutdrucksenkenden und positiv inotropen Wirkung an der narkotisierten Katze:

Die Untersuchungen wurden an Katzen durchgeführt, die mit Pentobarbital-Natrium (40 mg/kg i.p. + 8 mg/kg/Stunde) narkotisiert waren. Die Tiere atmeten spontan. Der arterielle Blutdruck wurde in der Aorta abdominalis mit einem StathamDruckwandler (P 23 Dc) gemessen. Für die Erfassung der positiv inotropen Wirkung wurde mit einem Kathetertipmanometer (Millar PC-350 A) der Druck in der linken Herzkammer gemessen. Daraus wurde der Kontraktilitätsparameter dp/dt $_{max}$ mittels eines Analogdifferenzierers gewonnen. Die zu untersuchende Substanz wurde in eine Vena femoralis injiziert. Als Lösungsmittel diente Polydiol 200. Die Substanz wurde an 2 Katzen geprüft, Dosis 0,3 mg/kg i.v..

Die nachfolgende Tabelle enthält die Mittelwerte:

| Substanz | Dosis mg/kg i. v. | diastol. Blutdruck änderung in mmHg | Zunahme von dp/dt in % | Wirkungsdauer (Halbwertszeit) | Zunahme der Herzfrequenz |
|---|---|---|---|---|---|
| A | 0,3 | -33 | +55 | >80 Minuten | + 9 % |
| B | 0,3 | -31 | +49 | 26 Minuten | + 9 % |
| C | 0,3 | -16 | +91 | >60 Minuten | +12 % |
| D | 0,3 | -31 | +50 | 7 Minuten | + 9 % |

In diesem Zusammenhang sei darauf hingewiesen, daß bei den biologischen Untersuchungen keine toxischen Nebenwirkungen der zu untersuchenden Substanz beobachtet wurden.

Aufgrund ihrer pharmakologischen Eigenschaften eignen sich die erfindungsgemäß hergestellten Verbindungen der Formel I und deren optisch aktive Antipoden sowie deren physiologisch verträgliche Säureadditionssalze mit anorganischen oder organischen Säuren zur Behandlung der chronischen und

akuten Herzinsuffizienz unterschiedlicher Genese und/oder zur Prophylaxe bzw. Behandlung von Thromboembolien und arteriellen Verschlußkrankheiten.

Hierzu lassen sich die neuen Verbindungen, gegebenenfalls in Kombination mit anderen Wirksubstanzen, in die üblichen pharmazeutischen Anwendungsformen wie Tabletten, Dragées, Pulver, Suspensionen, Suppositorien oder Ampullen einarbeiten. Die Einzeldosis beträgt hierbei beim Erwachsenen 1-4 $\times$ täglich bei intravenöser Applikation 1-50 mg, vorzugsweise 2 bis 40 mg, und bei oraler Applikation 5-150 mg, vorzugsweise 5 bis 100 mg.

Die nachfolgenden Beispiele sollen die Erfindung näher erläutern:

Beispiel A

5-Acetamido-1-äthyl-3,3-dimethyl-indolin-2-on

218,3 g (1 Mol) 5-Acetamido-3,3-dimethyl-indolin-2-on werden bei Raumtemperatur in 1 1 Dimethylformamid suspendiert und unter starkem Rühren mit 123,4 g (1,1 Mol) Kalium-tert.butylat portionsweise versetzt. Die Temperatur steigt dabei auf 38°C an. Nach halbstündigem Nachrühren, wenn die Temperatur auf 18-23°C abgesunken ist, tropft man innmerhalb 25 Minuten unter Rühren 81,6 ml = 119,8 g (1,1 Mol) Äthylbromid zu und rührt noch eine Stunde bei Raumtemperatur. Bei einer Badtemperatur von 50°C werden nunmehr im Wasserstrahlvakuum 800 ml Lösungsmittel abgezogen, der Rückstand mit 2,4 1 Eiswasser versetzt und eine Stunde gerührt. Das ausgefallene Produkt wird abgesaugt mit 1 1 destilliertem Wasser gewaschen und bei 50°C getrocknet.
Ausbeute: 197,1 g (80 % der Theorie),
Schmelzpunkt: 156-157°C

Analog wurden folgende Verbindungen hergestellt:

5-Acetamido-1,3,3-trimethyl-indolin-2-on
Schmelzpunkt: 159-161°C

5-Acetamido-1-benzyl-3,3-dimethyl-indolin-2-on
Schmelzpunkt: 169-170°C

5-Acetamido-3,3-dimethyl-1-isopropyl-indolin-2-on
Schmelzpunkt: 165-166°C

5-Acetamido-1-methyl-indolin-2-on
Schmelzpunkt: 246-248°C

5-Acetamido-1-äthyl-3-methyl-indolin-2-on
Schmelzpunkt: 149-150°C

5-Acetamido-3,3-dimethyl-1-n-propyl-indolin-2-on
Schmelzpunkt: 106-107°C

5-Acetamido-3,3-dimethyl-1-(2-methoxy-äthyl)-indolin-2-on

5-Acetamido-3,3-dimethyl-1-n-pentyl-indolin-2-on

5-Acetamido-1-n-butyl-3,3-dimethyl-indolin-2-on

5-(N-Methyl-acetamido)-1,3,3-trimethyl-indolin-2-on
Schmelzpunkt: 137°C

6-(N-Methyl-acetamido)-1,3,3-trimethyl-indolin-2-on
Schmelzpunkt: 139-140°C

Beispiel B

1-Äthyl-5-amino-3,3-dimethyl-6-nitro-indolin-2-on

197 g (0,8 Mol) 5-Acetamido-1-äthyl-3,3-dimethyl-indolin-2-on werden in 1,3 1 Eisessig gelöst und bei 18-20°C innerhalb 20 Minuten unter Rühren tropfenweise mit 80 ml rauchender Salpetersäure (d = 1,50) versetzt. Nach 10-minütigem Nachrühren wird mit Eiswasser auf ein Volumen von 10 1 verdünnt. Das ausgefallene gelbe 5-Acetamido-1-äthyl-3,3-dimethyl-6-nitro-indolin-2-on wird abgesaugt, mit Wasser gewaschen, mit 0,5 1 6 n Salzsäure, 0,5 1 Methanol und 0,5 1 Isopropanol versetzt und 2,5 Stunden zum Rückfluß erhitzt. Nach Abfiltrieren von etwas Ungelöstem über ein Schnellauffilter versetzt man das Filtrat mit 1 kg Eis, saugt das organgefarbene Produkt ab, wäscht es mit Wasser und trocknet es im Umlufttrockenschrank.
Ausbeute: 134 g (68 % der Theorie),
Schmelzpunkt: 187-188°C

Analog wurden folgende Verbindungen hergestellt:

5-Amino-3,3-dimethyl-1-(2-methoxy-äthyl)-6-nitro-indolin-2-on
Schmelzpunkt: 166-167°C

5-Amino-6-nitro-1,3,3-trimethyl-indolin-2-on
Schmelzpunkt: > 290°C

5-Amino-1-benzyl-3,3-dimethyl-6-nitro-indolin-2-on
Schmelzpunkt: 117°C

5-Amino-3,3-dimethyl-1-isopropyl-6-nitro-indolin-2-on
Schmelzpunkt: 205-206°C

5-Amino-3,3-dimethyl-6-nitro-1-n-pentyl-indolin-2-on
Schmelzpunkt: 113-114°C

5-Amino-1-methyl-6-nitro-indolin-2-on
Schmelzpunkt: 244-246°C

5-Amino-1-äthyl-3-methyl-6-nitro-indolin-2-on
Schmelzpunkt: 207-208°C

5-Amino-3,3-dimethyl-6-nitro-1-n-propyl-indolin-2-on
Schmelzpunkt: 186-187°C

5-Amino-1-n-butyl-3,3-dimethyl-6-nitro-indolin-2-on
Schmelzpunkt: 92-94°C

5-Methylamino-6-nitro-1,3,3-trimethyl-indolin-2-on
Schmelzpunkt: 236-237°C

6-Methylamino-5-nitro-1,3,3-trimethyl-indolin-2-on
Schmelzpunkt: 257-258°C

Beispiel C

1-Äthyl-5,6-diamino-3,3-dimethyl-indolin-2-on-dihydrochlorid

25 g (0,1 Mol) 1-Äthyl-5-amino-3,3-dimethyl-6-nitro-indolin-2-on werden in 250 ml Methanol und 40 ml methanolischer Salzsäure gelöst, mit 2,5 g 10%iger Palladiumkohle versetzt und während 2 Stunden bei Raumtemperatur hydriert. Man filtriert vom Katalysator ab, versetzt das Filtrat mit 2 1 Äther und kühlt auf -20°C ab. Den Niederschlag saugt man ab, wäscht ihn mit Äther und trocknet im Exsiccator.
Ausbeute: 28,5 g (97,5 % der Theorie),
Schmelzpunkt: > 300°C

Beispiel D

1-Äthyl-3,3-dimethyl-5-isonicotinoylamido-6-nitro-indolin-2-on

26,5 g (0,106 Mol) 1-Äthyl-5-amino-3,3-dimethyl-6-nitro-indolin-2-on werden in 300 ml Pyridin gelöst und bei Raumtemperatur portionsweise mit 26,7 g (0,15 Mol) Isonicotinsäurechlorid-hydrochlorid versetzt. Nach 20-stündigem Rühren wird langsam mit Wasser verdünnt. Der gelbe Niederschlag wird abgesaugt, mit 500 ml Wasser, 150 ml Isopropanol und 200 ml Diisopropyläther gewaschen und getrocknet.
Ausbeute: 30,2 g (80,5 % der Theorie),
Schmelzpunkt: 192°C

Analog - oder auch durch Erhitzen der entsprechenden Säurechloride mit den entsprechenden Amino-nitro-indolin-2-onen in Chlorbenzol auf 80-140°C - wurden folgende Verbindungen hergestellt:

5-Isonicotinoylamido-6-nitro-1,3,3-trimethyl-indolin-2-on
Schmelzpunkt: 250-252°C

1-Äthyl-3,3-dimethyl-5-(4-methoxy-benzoylamido)-6-nitro-indolin-2-on
Schmelzpunkt: 158-159°C

1-Äthyl-3,3-dimethyl-5-nicotinoylamido-6-nitro-indolin-2-on
Schmelzpunkt: 161-162°C

5-(4-Methoxy-benzoylamido)-6-nitro-1,3,3-trimethyl-indolin-2-on
Schmelzpunkt: 217-218°C

5-Nicotinoylamido-6-nitro-1,3,3-trimethyl-indolin-2-on
Schmelzpunkt: 209°C

3,3-Dimethyl-1-(2-methoxy-äthyl)-6-nitro-5-picoloylamido-indolin-2-on
Schmelzpunkt: 160-161°C

3,3-Dimethyl-1-(2-methoxy-äthyl)-5-nicotinoylamido-6-nitro-indolin-2-on
Schmelzpunkt: 132-133°C

3,3-Dimethyl-1-(2-methoxy-äthyl)-5-(4-methoxy-benzoylamido)-6-nitro-indolin-2-on
Schmelzpunkt: 158-159°C

3,3-Dimethyl-5-isonicotinoylamido-6-nitro-indolin-2-on
Schmelzpunkt: 130-131°C

1-Benzyl-3,3-dimethyl-5-isonicotinoylamido-6-nitro-indolin-2-on
Schmelzpunkt: 162-163°C

1-Äthyl-5-isonicotinoylamido-3-methyl-6-nitro-indolin-2-on
Schmelzpunkt: 170-171°C

1-Methyl-5-nicotinoylamido-6-nitro-indolin-2-on
Schmelzpunkt: 209°C

1-Benzyl-3,3-dimethyl-5-(4-methoxy-benzoylamido)-6-nitro-indolin-2-on
Schmelzpunkt: 147-149°C

6-Nitro-5-(3-phenyl-propionamido)-1,3,3-trimethyl-indolin-2-on
Schmelzpunkt: 138-139°C

6-Nitro-5-phenylacetamido-1,3,3-trimethyl-indolin-2-on
Schmelzpunkt: 138-140°C

3,3-Dimethyl-1-isopropyl-5-nicotinoylamido-6-nitro-indolin-2-on
Schmelzpunkt: 164-165°C

3,3-Dimethyl-1-isopropyl-5-(4-methoxy-benzoylamido)-6-nitro-indolin-2-on
Schmelzpunkt: 134-136°C

3,3-Dimethyl-5-nicotinoylamido-6-nitro-1-n-pentyl-indolin-2-on
Schmelzpunkt: 164-165°C

3,3-Dimethyl-5-(4-methoxy-benzoylamido)-6-nitro-1-n-pentyl-indolin-2-on
Schmelzpunkt: 143-145°C

3,3-Dimethyl-5-isonicotinoylamido-1-isopropyl-6-nitro-indolin-2-on
Schmelzpunkt: 165-166°C

3,3-Dimethyl-5-isonicotinoylamido-6-nitro-1-n-pentyl-indolin-2-on
Schmelzpunkt: 143-144°C

1-Äthyl-5-(3,5-di-tert.butyl-4-hydroxy-benzoylamido)-3,3-dimethyl-6-nitro-indolin-2-on
Schmelzpunkt: 247-248°C

1-n-Butyl-3,3-dimethyl-5-nicotinoylamido-6-nitro-indolin-2-on
Schmelzpunkt: 113-114°C

3,3-Dimethyl-5-(4-methoxy-benzoylamido)-6-nitro-1-n-propyl-indolin-2-on
Schmelzpunkt: 188-189°C

1-n-Butyl-3,3-dimethyl-5-(4-methoxy-benzoylamido)-6-nitro-indolin-2-on
Schmelzpunkt: 175-176°C

1-n-Butyl-3,3-dimethyl-5-isonicotinoylamido-6-nitro-indolin-2-on
Schmelzpunkt: 133-134°C

1-Äthyl-3,3-dimethyl-6-nitro-5-(3-thenoylamido)-indolin-2-on
Schmelzpunkt: 202-203°C

1-Äthyl-3,3-dimethyl-5-(3-furoylamido)-6-nitro-indolin-2-on
Schmelzpunkt: 193-194°C

3,3-Dimethyl-5-(3-furoylamido)-1-isoproyl-6-nitro-indolin-2-on
Schmelzpunkt: 148-149°C

3,3-Dimethyl-5-(3-furoylamido)-6-nitro-1-n-pentyl-indolin-2-on
Schmelzpunkt: 103-104°C

1-Äthyl-5-(4-amino-3,5-dibrom-benzoylamido)-3,3-dimethyl-6-nitro-indolin-2-on
Schmelzpunkt: 216-218°C

3,3-Dimethyl-5-nicotinoylamido-6-nitro-1-n-propyl-indolin-2-on
Schmelzpunkt: 147°C

3,3-Dimethyl-5-isonicotinoylamido-6-nitro-1-n-propyl-indolin-2-on
Schmelzpunkt: 137°C

3,3-Dimethyl-5-(3-furoylamido)-1-(2-methoxy-äthyl)-6-nitro-indolin-2-on
Schmelzpunkt: 128-129°C

3,3-Dimethyl-1-isopropyl-6-nitro-5-(3-thenoylamido)-indolin-2-on

Schmelzpunkt: 153°C

3,3-Dimethyl-1-n-pentyl-6-nitro-5-(3-thenoylamido)-indolin-2-on
Schmelzpunkt: 115-116°C

3,3-Dimethyl-1-(2-methoxy-äthyl)-6-nitro-5-(3-thenoylamido)-indolin-2-on
Schmelzpunkt: 133-134°C

1-Äthyl-3,3-dimethyl-6-nitro-5-(2-pyrazinoylamido)-indolin-2-on
Schmelzpunkt: 217°C

1-Äthyl-3,3-dimethyl-5-(2-furoylamido)-6-nitro-indolin-2-on
Schmelzpunkt: 167°C

1-Äthyl-3,3-dimethyl-6-nitro-5-(2-thenoylamido)-indolin-2-on
Schmelzpunkt: 177°C

1-Äthyl-3,3-dimethyl-5-(4-methylmercapto-benzoylamido)-6-nitro-indolin-2-on
Schmelzpunkt: 148-150°C

1-Äthyl-3,3-dimethyl-5-(4-dimethylamino-benzoylamido)-6-nitro-indolin-2-on
Schmelzpunkt: 164-166°C

1-Äthyl-3,3-dimethyl-5-(2-methoxy-4-methylmercapto-benzoylamido)-6-nitro-indolin-2-on
Schmelzpunkt: 219°C

1-Äthyl-5-(2-chinolin-carbonamido)-3,3-dimethyl-6-nitro-indolin-2-on
Schmelzpunkt: 220-222°C

1-Äthyl-5-(4-amino-benzoylamido)-3,3-dimethyl-6-nitro-indolin-2-on
Schmelzpunkt: 180°C

5-(1-Acetyl-4-piperidin-carbonamido)-1-äthyl-3,3-dimethyl-6-nitro-indolin-2-on
Schmelzpunkt: 178°C

1-Äthyl-5-(4-chlor-benzoylamido)-3,3-dimethyl-6-nitro-indolin-2-on
Schmelzpunkt: 170-172°C

1-Äthyl-5-(4-chinolin-carbonamido)-3,3-dimethyl-6-nitro-indolin-2-on
Schmelzpunkt: 239°C

1-Äthyl-5-(4-chinolin-carbonamido)-3,3-dimethyl-6-nitro-indolin-2-on
Schmelzpunkt: 239°C

1-Äthyl-5-(4-chinolin-carbonamido)-3,3-dimethyl-6-nitro-indolin-2-on
Schmelzpunkt: 239°C

1-Äthyl-3,3-dimethyl-5-(2-dimethylamino-nicotinoylamido)-6-nitro-indolin-2-on
Schmelzpunkt: 140-142°C

1-Äthyl-3,3-dimethyl-5-(2-morpholino-nicotinoylamido)-6-nitro-indolin-2-on
Schmelzpunkt: 167°C

1-Äthyl-5-(2,6-dichlor-isonicotinoylamido)-3,3-dimethyl-6-nitro-indolin-2-on
Schmelzpunkt: 197°C

1-Äthyl-5-(2-chlor-6-morpholino-isonicotinoylamido)-3,3-dimethyl-6-nitro-indolin-2-on
Schmelzpunkt: 208°C

1-Äthyl-3,3-dimethyl-5-(pyrimidin-5-carbonamido)-6-nitro-indolin-2-on
Schmelzpunkt: 215°C

3,3-Dimethyl-1-n-propyl-6-nitro-5-(2-thenoylamido)-indolin-2-on
Schmelzpunkt: 140°C

3,3-Dimethyl-5-(2-furoylamido)-6-nitro-1-n-propyl-indolin-2-on
Schmelzpunkt: 187°C

3,3-Dimethyl-5-(4-methylmercapto-benzoylamido)-6-nitro-1-n-propyl-indolin-2-on
Schmelzpunkt: 156-158°C

3,3-Dimethyl-6-nitro-1-n-pentyl-5-(2-thenoylamido)-indolin-2-on
Schmelzpunkt: 125-127°C

1-Äthyl-5-(4-cyan-benzoylamido)-3,3-dimethyl-6-nitro-indolin-2-on
Schmelzpunkt: 197-199°C

1-Äthyl-5-(2-chlor-benzoylamido)-3,3-dimethyl-6-nitro-indolin-2-on
Schmelzpunkt: 169°C

5-(2-Phenyl-acrylamido)-6-nitro-1,3,3-trimethyl-indolin-2-on
Schmelzpunkt: 209-211°C

6-Nitro-5-(2-pyrazinoylamido)-1,3,3-trimethyl-indolin-2-on
Schmelzpunkt: > 280°C

6-Nitro-5-(2-thenoylamido)-1,3,3-trimethyl-indolin-2-on
Schmelzpunkt: 220°C

3,3-Dimethyl-6-nitro-1-n-propyl-5-(2-pyrazinoylamido)-indolin-2-on
Schmelzpunkt: 185°C

1-Äthyl-3,3-dimethyl-5-(4-methyl-5-oxazolyl-carbonamido)-6-nitro-indolin-2-on
Schmelzpunkt: 230°C

1-Äthyl-5-(4-amino-3,5-dichlor-benzoylamido)-3,3-dimethyl-6-nitro-indolin-2-on
Schmelzpunkt: 226-228°C

1-Äthyl-3,3-dimethyl-6-nitro-5-(pyrimidin-4-carbonamido)-indolin-2-on
Schmelzpunkt: 208°C

3,3-Dimethyl-6-nitro-1-n-pentyl-5-(2-pyrazinoylamido)-indolin-2-on
Schmelzpunkt: 138°C

3,3-Dimethyl-5-(2-furoylamido)-6-nitro-1-n-pentyl-indolin-2-on
Schmelzpunkt: 135°C

6-Nitro-5-(N-methyl-isonicotinoylamido)-1,3,3-trimethyl-indolin-2-on
Schmelzpunkt: 190-192°C

5-Nitro-6-(N-methyl-isonicotinoylamido)-1,3,3-trimethyl-indolin-2-on
Schmelzpunkt: 188-189°C

5-Isonicotinoylamido-1-methyl-6-nitro-indolin-2-on
Schmelzpunkt: 239-240°C

3,3-:í-5-(4-methylmercapto-benzoylamido)-6-nitro-1-n-pentyl-indolin-2-on
Schmelzpunkt: 134°C

3,3-Dimethyl-1-(2-methoxy-äthyl)-5-(5-methyl-3-furoylamido)-6-nitro-indolin-2-on
Schmelzpunkt: 144°C

1-Äthyl-3,3-dimethyl-5-(5-methyl-3-furoylamido)-6-nitro-indolin-2-on
Schmelzpunkt: 152-154°C

3,3-Dimethyl-1-isopropyl-5-(5-methyl-3-furoylamido)-6-nitro-indolin-2-on
Schmelzpunkt: 155°C

3,3-Dimethyl-5-(5-methyl-3-furoylamido)-6-nitro-1-n-pentyl-indolin-2-on
Schmelzpunkt: 93-95°C

1-Äthyl-5-benzoylamido-3,3-dimethyl-6-nitro-indolin-2-on
Schmelzpunkt: 172-173°C

1-Äthyl-5-butyrylamido-3,3-dimethyl-6-nitro-indolin-2-on
Schmelzpunkt: 99-100°C

1-Äthyl-3,3-dimethyl-6-nitro-5-(2-thienyl-acetamido)-indolin-2-on
Schmelzpunkt: 158°C

1-Äthyl-3,3-dimethyl-6-nitro-5-(3-thienyl-acetamido)-indolin-2-on
Schmelzpunkt: 160°C

1-n-Butyl-3,3-dimethyl-6-nitro-5-(2-thenoylamido)-indolin-2-on
Schmelzpunkt: 143°C

1-n-Butyl-3,3-dimethyl-6-nitro-5-(3-thienyl-acetamido)-indolin-2-on
Öl, Rf-Wert: 0,61 (Kieselgel, Äthylenchlorid/Aceton = 20:1)

1-n-Butyl-3,3-dimethyl-6-nitro-5-(2-thienyl-acetamido)-indolin-2-on
Öl, Rf-Wert: 0,62 (Kieselgel, Äthylenchlorid/Aceton = 20:1)

## Beispiel 1

### 2-(4-Pyridyl)-5,7,7-trimethyl-6,7-dihydro-3H,5H-pyrrolo[2,3-f]benzimidazol-6-on

1,95 g (5,7 mMol) 5-Isonicotinoylamido-6-nitro-1,3,3-trimethyl-indolin-2-on werden in 60 ml Eissessig gelöst, mit 1 g Raney-Nickel versetzt und während 4 Stunden bei 80°C und 5 bar Wasserstoffdruck im Autoklaven hydriert. Nachdem man vom Katalysator abfiltriert hat, wird das Filtrat zur Trokkene eingeengt, mit Äthanol/Wasser aufgerührt, mit wässrigem Ammoniak alkalisch gestellt und abgesaugt.
Ausbeute: 1 g (58,5 % der Theorie),
Schmelzpunkt: ab 161°C (Zers.; 30%iges Äthanol)
$C_{17}H_{16}N_4O \times 0,4\ H_2O$ (299,55)
Ber: C 68,17 H 5,65 N 18,70
Gef: C 68,13 H 5,77 N 19,00

## Beispiel 2

5-Äthyl-7,7-dimethyl-2-(4-pyridyl)-6,7-dihydro-3H,5H-pyrrolo[2,3-f]benzimidazol-6-on

Hergestellt analog Beispiel 1 aus 1-Äthyl-3,3-dimethyl-5-isonicotinoylamido-6-nitro-indolin-2-on und Wasserstoff/Raney-Nickel.
Ausbeute: 55 % der Theorie,
Schmelzpunkt: 297-298°C (Isopropanol/Diisopropyläther)
$C_{18}H_{18}N_4O$ (306,37)
Ber: C 70,57 H 5,92 N 18,29
Gef: C 70,60 H 6,18 N 18,10

Beispiel 3

5-Äthyl-7,7-dimethyl-2-(4-methoxy-phenyl)-6,7-dihydro-3H,5Hpyrrolo[2,3-f]benzimidazol-6-on

Hergestellt analog Beispiel 1 aus 1-Äthyl-3,3-dimethyl-5-(4-methoxy-benzoylamino)-6-nitro-indolin-2-on und Wasserstoff/Raney-Nickel.
Ausbeute: 78 % der Theorie,
Schmelzpunkt: 275-277°C (Isopropanol/Diäthyläther)
$C_{20}H_{12}N_3O_2$ (335,41)
Ber: C 71,62 H 6,31 N 12,53
Gef: C 71,83 H 6,48 N 12,65

Beispiel 4

5-Äthyl-7,7-dimethyl-2-(3-pyridyl)-6,7-dihydro-3H,5H-pyrrolo[2,3-f]benzimidazol-6-on

Hergestellt analog Beispiel 1 aus 1-Äthyl-3,3-dimethyl-5-nicotinoylamido-6-nitro-indolin-2-on und Wasserstoff/RaneyNickel.
Ausbeute: 45 % der Theorie,
Schmelzpunkt: 299-300°C (Isopropanol/Diisopropyläther)
$C_{18}H_{18}N_4O$ (306,37)
Ber: C 70,57 H 5,92 N 18,29
Gef: C 70,70 H 6,14 N 18,42

Beispiel 5

2-(4-Methoxy-phenyl)-5,7,7-trimethyl-6,7-dihydro-3H,5H-pyrrolo[2,3-f]benzimidazol-6-on

Hergestellt analog Beispiel 1 aus 5-(4-Methoxy-benzoylamino)-6-nitro-1,3,3-trimethyl-indolin-2-on und Wasserstoff/Raney-Nickel.
Ausbeute: 60 % der Theorie,
Schmelzpunkt: 217-218°C (Essigester/Diisopropyläther)
$C_{19}H_{19}N_3O_2$ (321,38)
Ber: C 71,00 H 5,96 N 13,08
Gef: C 70,75 H 5,94 N 12,98

Beispiel 6

2-(3-Pyridyl)-5,7,7-trimethyl-6,7-dihydro-3H,5H-pyrrolo[2,3-f]benzimidazol-6-on x 0,5 $H_2O$

Hergestellt analog Beispiel 1 aus 5-Nicotinoylamido-6-nitro-1,3,3-trimethyl-indolin-2-on und Wasserstoff/Raney-Nickel.
Ausbeute: 66 % der Theorie,

Schmelzpunkt: 297-298°C (Essigester/Diisopropyläther)
$C_{17}H_{16}N_4O \times 0,5\ H_2O$ (301,35)
Ber: C 67,75 H 5,68 N 18,59
Gef: C 67,58 H 5,73 N 18,40

Beispiel 7

7,7-Dimethyl-5-(2-methoxy-äthyl)-2-(2-pyridyl)-6,7-dihydro-3H,5H-pyrrolo[2,3-f]benzimidazol-6-on

Hergestellt analog Beispiel 1 aus 3,3-Dimethyl-1-(2-methoxy-äthyl)-6-nitro-5-picoloylamino-indolin-2-on und Wasserstoff/Raney-Nickel.
Ausbeute: 28 % der Theorie,
Schmelzpunkt: 216-217°C (Essigester/Diisopropyläther)
$C_{19}H_{20}N_4O_2$ (336,40)
Ber: C 67,84 H 5,99 N 16,66
Gef: C 68,10 H 6,05 N 16,49

Beispiel 8

7,7-Dimethyl-5-(2-methoxy-äthyl)-2-(3-pyridyl)-6,7-dihydro-3H,5H-pyrrolo[2,3-f]benzimidazol-6-on

Hergestellt analog Beispiel 1 aus 3,3-Dimethyl-1-(2-methoxy-äthyl)-5-nicotinoylamido-6-nitro-indolin-2-on und Wasserstoff/Raney-Nickel.
Ausbeute: 39 % der Theorie,
Schmelzpunkt: 202-203°C (Essigester/Diisopropyläther)
$C_{19}H_{20}N_4O_2$ (336,40)
Ber: C 67,84 H 5,99 N 16,66
Gef: C 68,21 H 6,13 N 16,55

Beispiel 9

7,7-Dimethyl-5-(2-methoxy-äthyl)-2-(4-methoxy-phenyl)-6,7-dihydro-3H,5H-pyrrolo[2,3-f]benzimidazol-6-on

Hergestellt analog Beispiel 1 aus 3,3-Dimethyl-1-(2-meth oxy-äthyl)-5-(4-methoxy-benzoylamino)-6-nitro-indolin-2-on und Wasserstoff/Raney-Nickel.
Ausbeute: 78,8 % der Theorie,
Schmelzpunkt: 232-233°C (Essigester/Diisopropyläther)
$C_{21}H_{23}N_3O_3$ (365,44)
Ber: C 69,02 H 6,34 N 11,50
Gef: C 69,33 H 6,36 N 11,75

Beispiel 10

2-(4-Hydroxy-phenyl)-5,7,7-trimethyl-6,7-dihydro-3H,5H-pyrrolo[2,3-f]benzimidazol-6-on $\times$ 0,5 $H_2O$

1,5 g (4,6 mMol) 2-(4-Methoxy-phenyl)-5,7,7-trimethyl-6,7-dihydro-3H,5H-pyrrolo[2,3-f]benzimidazol-6-on werden in 100 ml wasserfreiem Äthylenchlorid gelöst und bei -20°C mit 1,17 g (4,6 mMol) Bortribromid versetzt. Nach dreitägigem Rühren bei Raumtemperatur werden weitere 1,17 g Bortribromid zugegeben und weitere 2 Tage bei Raumtemperatur gerührt. Danach wird die Reaktionsmischung mit Äthanol/Wasser = 1:1 zersetzt, der nach dem Abdestillieren der Lösungsmittel verbleibende Rückstand in verdünnter Natronlauge gelöst und mit Chloroform/Äthanol = 4:1 extrahiert. Die wässrig-alkalische Phase wird mit Aktivkohle versetzt und nach einiger Zeit von der Aktivkohle abfiltriert. Das Filtrat wird durch Zugabe von verdünnter Essigsäure auf pH = 6 gestellt, der Niederschlag abgesaugt, mit Wasser gewaschen und bei 60°C getrocknet.

Ausbeute: 1,03 g (69 % der Theorie),
Schmelzpunkt: 211 °C
$C_{18}H_{17}N_3O_2 \times 0,5\ H_2O$ (316,37)
Ber: C 68,34 H 5,74 N 13,28
Gef: C 68,61 H 5,55 N 13,27

Beispiel 11

5-Äthyl-7,7-dimethyl-2-(4-hydroxy-phenyl)-6,7-dihydro-3H,5H-pyrrolo[2,3-f]benzimidazol-6-on × 2 $H_2O$

Eine Mischung von 1,7 g (5 mMol) 5-Äthyl-7,7-dimethyl-2-(4-methoxy-phenyl)-6,7-dihydro-3H,5H-pyrrolo[2,3-f]benzimidazol-6-on und 17 g Pyridin-hydrochlorid wird 4 Stunden lang bei 180°C gerührt. Nach dem Abkühlen wird mit Wasser versetzt und vom Ungelösten abfiltriert. Der erhaltene Rückstand wird in 0,1 n Natronlauge gelöst und mit Chloroform/Methanol (4:1) extrahiert. Die wässrig-alkalische Phase wird mit Aktivkohle versetzt und nach einiger Zeit abfiltriert. Durch Zugabe von verdünnter Essigsäure bringt man das Filtrat auf pH 5-6. Der ausgefallene Niederschlag wird abgesaugt, mit Wasser gewaschen und getrocknet.
Ausbeute: 1,5 g (82,8 % der Theorie),
Schmelzpunkt: ab 295°C
$C_{19}H_{19}N_3O_2 \times 2\ H_2O$ (357,42)
Ber: C 63,85 H 6,49 N 11,76
Gef: C 63,85 H 6,27 N 12,01

Beispiel 12

7,7-Dimethyl-5-(2-methoxy-äthyl)-2-(4-pyridyl)-6,7-dihydro-3H,5H-pyrrolo[2,3-f]benzimidazol-6-on

Hergestellt analog Beispiel 1 aus 3,3-Dimethyl-5-isonicotinoylamido-6-nitro-indolin-2-on.
Ausbeute: 72 % der Theorie,
Schmelzpunkt: 255-256°C (Essigester)
$C_{19}H_{20}N_4O_2$ (336,40)
Ber.: C 67,84 H 5,99 N 16,66
Gef.: C 67,90 H 5,78 N 16,48

Beispiel 13

5-Benzyl-7,7-dimethyl-2-(4-pyridyl)-6,7-dihydro-3H,5H-pyrrolo[2,3-f]benzimidazol-6-on × 0,25 $H_2O$

Hergestellt analog Beispiel 1 aus 1-Benzyl-3,3-dimethyl-5-isonicotinoylamido-6-nitro-indolin-2-on.
Ausbeute: 78 % der Theorie,
Schmelzpunkt: ab 131°C (Essigester)
$C_{23}H_{20}N_4O \times 0,25\ H_2O$ (368,44)
Ber.: C 74,04 H 5,54 N 15,02
Gef.: C 74,10 H 5,68 N 15,02

Beispiel 14

5-Äthyl-7-methyl-2-(4-pyridyl)-6,7-dihydro-3H,5H-pyrrolo[2,3-f]benzimidazol-6-on

Hergestellt analog Beispiel 1 aus 1-Äthyl-5-isonicotinoylamido-3-methyl-6-nitro-indolin-2-on.
Ausbeute: 39 % der Theorie,
Schmelzpunkt: 264°C (Essigester/Isopropanol)
$C_{17}H_{16}N_4O$ (292,34)
Ber.: C 69,85 H 5,52 N 19,17
Gef.: C 69,85 H 5,61 N 19,30

Beispiel 15

5-Methyl-2-(3-pyridyl)-6,7-dihydro-3H,5H-pyrrolo[2,3-f]benzimidazol-6-on × 0,5 $H_2O$

Hergestellt analog Beispiel 1 aus 1-Methyl-5-nicotinoylamido-6-nitro-indolin-2-on.
Ausbeute: 58 % der Theorie,
Schmelzpunkt: > 295°C (Isopropanol/Diisopropyläther)
$C_{15}H_{12}N_4O$ × 0,5 $H_2O$ (273,29)
Ber.: C 65,92 H 4,79 N 20,50
Gef.: C 65,95 H 4,88 N 20,72

Beispiel 16

5-Benzyl-7,7-dimethyl-2-(4-methoxy-phenyl)-6,7-dihydro-3H,5H-pyrrolo[2,3-f]benzimidazol-6-on

Hergestellt analog Beispiel 1 aus 1-Benzyl-3,3-dimethyl-5-(4-methoxy-benzoylamido)-6-nitro-indolin-2-on.
Ausbeute: 68 % der Theorie,
Schmelzpunkt: 298-300°C (Isopropanol)
$C_{25}H_{23}N_3O_2$ (397,48)
Ber.: C 75,55 H 5,83 N 10,57
Gef.: C 75,74 H 5,90 N 10,57

Beispiel 17

2-(2-Phenyl-äthyl)-5,7,7-trimethyl-6,7-dihydro-3H,5H-pyrrolo[2,3-f]benzimidazol-6-on

Hergestellt analog Beispiel 1 aus 6-Nitro-5-(3-phenyl-propionamido)-1,3,3-trimethyl-indolin-2-on.
Ausbeute: 76 % der Theorie,
Schmelzpunkt: ab 138°C (Zers.; Cyclohexan/Essigester)
$C_{20}H_{21}N_3O$ (319,41)
Ber.: C 75,21 H 6,63 N 13,16
Gef.: C 75,36 H 6,87 N 12,91

Beispiel 18

2-Benzyl-5,7,7-trimethyl-6,7-dihydro-3H,5H-pyrrolo[2,3-f]benzimidazol-6-on

Hergestellt analog Beispiel 1 aus 6-Nitro-5-phenylacetamido-1,3,3-trimethyl-indolin-2-on.
Ausbeute: 72 % der Theorie,
Schmelzpunkt: 114-115°C (Essigester/Diisopropyläther)
$C_{19}H_{19}N_3O$ (305,38)
Ber.: C 74,73 H 6,27 N 13,76
Gef.: C 74,55 H 6,01 N 13,95

Beispiel 19

7,7-Dimethyl-2-(4-hydroxy-phenyl)-5-(2-pyridinium-äthyl)-6,7-dihydro-3H,5H-pyrrolo[2,3-f]benzimidazol-6-on x 1,5 $H_2O$

Hergestellt analog Beispiel 11 aus 7,7-Dimethyl-5-(2-methoxyäthyl)-2-(4-methoxy-phenyl)-6,7-dihydro-3H,5H-pyrrolo[2,3-f]benzimidazol-6-on und Pyridinhydrochlorid.
Ausbeute: 71 % der Theorie,
Schmelzpunkt: > 300°C (Wasser)
$C_{24}H_{23}ClN_4O_2$ x 1,5 $H_2O$ (461,95)
Ber.: C 62,40 H 5,67 N 12,13
Gef.: C 62,38 H 5,50 N 12,37

Beispiel 20

7,7-Dimethyl-5-isopropyl-2-(3-pyridyl)-6,7-dihydro-3H,5H-pyrrolo[2,3-f]benzimidazol-6-on

Hergestellt analog Beispiel 1 aus 3,3-Dimethyl-1-isopropyl-5-nicotinoylamido-6-nitro-indolin-2-on.
Ausbeute: 37,6 % der Theorie,
Schmelzpunkt: 224°C (Essigester/Diisopropyläther)
$C_{19}H_{20}N_4O$ (320,40)
Ber.: C 71,23 H 6,29 N 17,49
Gef.: C 71,05 H 6,41 N 17,37

Beispiel 21

7,7-Dimethyl-5-isopropyl-2-(4-methoxy-phenyl)-6,7-dihydro-3H,5H-pyrrolo[2,3-f]benzimidazol-6-on

Hergestellt analog Beispiel 1 aus 3,3-Dimethyl-1-isopropyl-5-(4-methoxy-benzoylamido)-6-nitro-indolin-2-on.
Ausbeute: 66,5 % der Theorie,
Schmelzpunkt: > 290°C (Essigester/Isopropanol)
$C_{21}H_{23}N_3O_2$ (349,43)
Ber.: C 72,18 H 6,63 N 12,03
Gef.: C 72,22 H 6,72 N 11,93

Beispiel 22

7,7-Dimethyl-5-n-pentyl-2-(3-pyridyl)-6,7-dihydro-3H,5H-pyrrolo[2,3-f]benzimidazol-6-on

Hergestellt analog Beispiel 1 aus 3,3-Dimethyl-5-nicotinoylamido-6-nitro-1-n-pentyl-indolin-2-on.
Ausbeute: 50 % der Theorie,
Schmelzpunkt: 177-178°C (Essigester/Diisopropyläther)
$C_{21}H_{24}N_4O$ (348,45)
Ber.: C 72,39 H 6,94 N 16,08
Gef.: C 72,31 H 6,92 N 15,96

Beispiel 23

7,7-Dimethyl-2-(4-methoxy-phenyl)-5-n-pentyl-6,7-dihydro-3H,5H-pyrrolo[2,3-f]benzimidazol-6-on

Hergestellt analog Beispiel 1 aus 3,3-Dimethyl-5-(4-methoxy-benzoylamido)-6-nitro-1-n-pentyl-indolin-2-on.
Ausbeute: 81 % der Theorie,

Schmelzpunkt: 213-214°C (Essigester)
$C_{23}H_{27}N_3O_2$ (477,49)
Ber.: C 73,18 H 7,21 N 11,13
Gef.: C 73,32 H 7,26 N 10,94

Beispiel 24

7,7-Dimethyl-5-isopropyl-2-(4-pyridyl)-6,7-dihydro-3H,5H-pyrrolo[2,3-f]benzimidazol-6-on

Hergestellt analog Beispiel 1 aus 3,3-Dimethyl-5-isonicotinoylamido-1-isopropyl-6-nitro-indolin-2-on.
Ausbeute: 56 % der Theorie,
Schmelzpunkt: 276-277°C (Essigester/Diisopropyläther)
$C_{19}H_{20}N_4O$ (320,40)
Ber.: C 71,23 H 6,29 N 17,49
Gef.: C 71,18 H 6,28 N 17,30

Beispiel 25

7,7-Dimethyl-5-n-pentyl-2-(4-pyridyl)-6,7-dihydro-3H,5H-pyrrolo[2,3-f]benzimidazol-6-on

Hergestellt analog Beispiel 1 aus 3,3-Dimethyl-5-isonicotinoylamido-6-nitro-1-n-pentyl-indolin-2-on.
Ausbeute: 78 % der Theorie,
Schmelzpunkt: 105-106°C (Essigester/Diisopropyläther)
$C_{21}H_{24}N_4O$ (348,45)
Ber.: C 72,39 H 6,94 N 16,08
Gef.: C 72,39 H 6,90 N 15,92

Beispiel 26

5-Äthyl-2-(3,5-di-tert.butyl-4-hydroxy-phenyl)-7,7-dimethyl-6,7-dihydro-3H,5H-pyrrolo[2,3-f]benzimidazol-6-on

Hergestellt analog Beispiel 1 aus 1-Äthyl-5-(3,5-di-tert.butyl-4-hydroxy-benzoylamido)-3,3-dimethyl-6-nitro-indolin-2-on.
Ausbeute: 40 % der Theorie,
Schmelzpunkt: > 300°C (Essigester/Diisopropyläther)
$C_{27}H_{35}N_3O_2$ (433,59)
Ber.: C 74,79 H 8,14 N 9,69
Gef.: C 74,73 H 8,33 N 9,50

Beispiel 27

5-n-Butyl-7,7-dimethyl-2-(3-pyridyl)-6,7-dihydro-3H,5H-pyrrolo[2,3-f]benzimidazol-6-on

Hergestellt analog Beispiel 1 aus 1-n-Butyl-3,3-dimethyl-5-nicotinoylamido-6-nitro-indolin-2-on.
Ausbeute: 64 % der Theorie,
Schmelzpunkt: 175-176°C (Essigester/Diisopropyläther)
$C_{20}H_{22}N_4O$ (334,42)
Ber.: C 71,83 H 6,63 N 16,75
Gef.: C 71,93 H 6,67 N 16,82

Beispiel 28

7,7-Dimethyl-2-(4-methoxy-phenyl)-5-n-propyl-6,7-dihydro-3H,5H-pyrrolo[2,3-f]benzimidazol-6-on

Hergestellt analog Beispiel 1 aus 3,3-Dimethyl-5-(4-methoxy-benzoylamido)-6-nitro-1-n-propyl-indolin-2-on.
Ausbeute: 74 % der Theorie,
Schmelzpunkt: 262-263°C (Essigester/Diisopropyläther)
$C_{21}H_{23}N_3O_2$ (349,43)
Ber.: C 72,18 H 6,63 N 12,03
Gef.: C 72,03 H 6,69 N 11,99

Beispiel 29

5-n-Butyl-7,7-dimethyl-2-(4-methoxy-phenyl)-6,7-dihydro-3H,5H-pyrrolo[2,3-f]benzimidazol-6-on

Hergestellt analog Beispiel 1 aus 1-n-Butyl-3,3-dimethyl-5-(4-methoxy-benzoylamido)-6-nitro-indolin-2-on.
Ausbeute: 70 % der Theorie,
Schmelzpunkt: 227-228°C (Essigester/Diisopropyläther)
$C_{22}H_{25}N_3O_2$ (363,46)
Ber.: C 72,70 H 6,93 N 11,56
Gef.: C 72,83 H 7,04 N 11,52

Beispiel 30

5-n-Butyl-7,7-dimethyl-2-(4-pyridyl)-6,7-dihydro-3H,5H-pyrrolo[2,3-f]benzimidazol-6-on

Hergestellt analog Beispiel 1 aus 1-n-Butyl-3,3-dimethyl-5-isonicotinoylamido-6-nitro-indolin-2-on.
Ausbeute: 67 % der Theorie,
Schmelzpunkt: 127-129°C (Essigester/Diisopropyläther)
$C_{20}H_{22}N_4O$ (334,42)
Ber.: C 71,83 H 6,63 N 16,75
Gef.: C 71,70 H 6,84 N 16,62

Beispiel 31

5-Äthyl-7,7-dimethyl-2-(3-thienyl)-6,7-dihydro-3H,5H-pyrrolo[2,3-f]benzimidazol-6-on

Hergestellt analog Beispiel 1 aus 1-Äthyl-3,3-dimethyl-6-nitro-5-(3-thenoylamido)-indolin-2-on.
Ausbeute: 52 % der Theorie,
Schmelzpunkt: > 295°C (Essigester/Isopropanol)
$C_{17}H_{17}N_3OS$ (311,41)
Ber.: C 65,57 H 5,50 N 13,49 S 10,30
Gef.: C 65,70 H 5,43 N 13,38 S 10,49

Beispiel 32

5-Äthyl-7,7-dimethyl-2-(3-furyl)-6,7-dihydro-3H,5H-pyrrolo[2,3-f]benzimidazol-6-on

Hergestellt analog Beispiel 1 aus 1-Äthyl-3,3-dimethyl-5-(3-furoylamido)-6-nitro-indolin-2-on.
Ausbeute: 65,2 % der Theorie,
Schmelzpunkt: 267-269°C (Essigester/Diisopropyläther)
$C_{17}H_{17}N_3O_2$ (295,34)
Ber.: C 69,14 H 5,80 N 14,23
Gef.: C 69,23 H 5,91 N 14,13

Beispiel 33:

7,7-Dimethyl-2-(3-furyl)-5-isopropyl-6,7-dihydro-3H,5H-pyrrolo[2,3-f]benzimidazol-6-on

Hergestellt analog Beispiel 1 aus 3.3-Dimethyl-5-(3-furoylamido)-1-isoproyl-6-nitro-indolin-2-on.
Ausbeute: 56 % der Theorie,
Schmelzpunkt: 282-284°C (Isopropanol/Essigester)
$C_{18}H_{19}N_3O_2$ (309,37)
Ber.: C 69,88 H 6,19 N 13,58
Gef.: C 69,66 H 6,25 N 13,31

Beispiel 34:

7,7-Dimethyl-2-(3-furyl)-5-n-pentyl-6,7-dihydro-3H.5H-pyrrolo[2,3-f]benzimidazol-6-on

Hergestellt analog Beispiel 1 aus 3,3-Dimethyl-5-(3-furoylamido)-6-nitro-1-n-pentyl-indolin-2-on.
Ausbeute: 70,4 % der Theorie,
Schmelzpunkt: 198°C (Essigester/Diisopropyläther)
$C_{20}H_{23}N_3O_2$ (337,42)
Ber.: C 70,37 H 6,98 N 11,84
Gef.: C 70,25 H 7,17 N 11,99

Beispiel 35

5-Äthyl-2-(4-amino-3,5-dibrom-phenyl)-7,7-dimethyl-6,7-dihydro-3H,5H-pyrrolo[2,3-f]benzimidazol-6-on

Hergestellt analog Beispiel 1 aus 1-Äthyl-5-(4-amino-3,5-dibrom-benzoylamido)-3,3-dimethyl-6-nitro-indolin-2-on.
Ausbeute: 28 % der Theorie,
Schmelzpunkt: > 300°C (Essigester/Äther)
$C_{19}H_{18}Br_2N_4O$ (478,21)
Ber.: C 47,72 H 3,79 N 11,72 Br 33,42
Gef.: C 47,54 H 3,86 N 11,58 33,46

Beispiel 36

7,7-Dimethyl-5-n-propyl-2-(3-pyridyl)-6,7-dihydro-3H,5H-pyrrolo[2,3-f]benzimidazol-6-on × 0,5 $H_2O$

Hergestellt analog Beispiel 1 aus 3,3-Dimethyl-5-nicotinoylamido-6-nitro-1-n-propyl-indolin-2-on.
Ausbeute: 54 % der Theorie,
Schmelzpunkt: ab 145°C (Zers.; Essigester/Diisopropyläther)
$C_{19}H_{20}N_4O$ × 0,5 $H_2O$ (329,40)
Ber.: C 69,28 H 6,42 N 17,01
Gef.: C 69,38 H 6,40 N 17,26

Beispiel 37

7,7-Dimethyl-5-n-propyl-2-(4-pyridyl)-6,7-dihydro-3H,5H-pyrrolo[2,3-f]benzimidazol-6-on

Hergestellt analog Beispiel 1 aus 3,3-Dimethyl-5-isonicotinoylamido-6-nitro-1-n-propyl-indolin-2-on.
Ausbeute: 58 % der Theorie,
Schmelzpunkt: > 295°C (Essigester/Diisopropyläther)
$C_{19}H_{18}Br_2N_4O$ (320,40)
Ber.: C 71,23 H 6,29 N 17,49
Gef.: C 71,15 H 6,49 N 17,60

Beispiel 38

7,7-Dimethyl-2-(3-furyl)-5-(2-methoxy-äthyl)-6,7-dihydro-3H,5H-pyrrolo[2,3-f]benzimidazol-6-on

Hergestellt analog Beispiel 1 aus 3,3-Dimethyl-5-(3-furoylamido)-1-(2-methoxy-äthyl)-6-nitro-indolin-2-on.
Ausbeute: 66,5 % der Theorie,
Schmelzpunkt: 226-227°C (Essigester/Diisopropyläther)
$C_{18}H_{19}N_3O_3$ (325,37)
Ber.: C 66,45 H 5,89 N 12,91
Gef.: C 66,45 H 5,96 N 12,70

Beispiel 39

7,7-Dimethyl-5-isopropyl-2-(3-thienyl)-6,7-dihydro-3H,5H-pyrrolo[2,3-f]benzimidazol-6-on

Hergestellt analog Beispiel 1 aus 3,3-Dimethyl-1-isopropyl-6-nitro-5-(3-thenoylamido)-indolin-2-on.
Ausbeute: 70,5 % der Theorie,
Schmelzpunkt: > 300°C (Essigester/Isopropanol)
$C_{18}H_{19}N_3OS$ (325,43)
Ber.: C 66,43 H 5,88 N 12,91 S 9,85
Gef.: C 66,54 H 6,07 N 12,70 S 9,66

Beispiel 40

7,7-Dimethyl-5-n-pentyl-2-(3-thienyl)-6,7-dihydro-3H,5H-pyrrolo[2,3-f]benzimidazol-6-on

Hergestellt analog Beispiel 1 aus 3,3-Dimethyl-1-n-pentyl-6-nitro-5-(3-thenoylamido)-indolin-2-on.
Ausbeute: 56,6 % der Theorie,
Schmelzpunkt: 163-164°C (Essigester/Diisopropyläther)
$C_{20}H_{23}N_3OS$ (353,48)
Ber.: C 67,98 H 6,56 N 11,89 S 9,07
Gef.: C 67,76 H 6,72 N 11,68 S 8,88

Beispiel 41

7,7-Dimethyl-5-(2-methoxy-äthyl)-2-(3-thienyl)-6,7-dihydro-3H,5H-pyrrolo[2,3-f]benzimidazol-6-on

Hergestellt analog Beispiel 1 aus 3,3-Dimethyl-1-(2-methoxy-äthyl)-6-nitro-5-(3-thenoylamido)-indolin-2-on.
Ausbeute: 57,6 % der Theorie,
Schmelzpunkt: 260-261°C (Essigester/Diisopropyläther)
$C_{18}H_{19}N_3O_2S$ (341,43)
Ber.: C 63,32 H 5,61 N 12,31 S 9,39
Gef.: C 63,51 H 5,74 N 12,10 S 9,19

Beispiel 42

5-Äthyl-7,7-dimethyl-2-(2-pyrazinyl)-6,7-dihydro-3H,5H-pyrrolo[2,3-f]benzimidazol-6-on × 0,5 $H_2O$

Hergestellt analog Beispiel 1 aus 1-Äthyl-3,3-dimethyl-6-nitro-5-(2-pyrazinoylamido)-indolin-2-on.
Ausbeute: 22,8 % der Theorie,
Schmelzpunkt: > 290°C
$C_{17}H_{17}N_5O$ × 0,5 $H_2O$ (316,36)
Ber.: C 64,54 H 5,73 N 22,14
Gef.: C 64,68 H 5,87 N 22,38

Beispiel 43

5-Äthyl-7,7-dimethyl-2-(2-furyl)-6,7-dihydro-3H,5H-pyrrolo[2,3-f]benzimidazol-6-on

Hergestellt analog Beispiel 1 aus 1-Äthyl-3,3-dimethyl-5-(2-furoylamido)-6-nitro-indolin-2-on.
Ausbeute: 44,3 % der Theorie,
Schmelzpunkt: > 290°C
$C_{17}H_{17}N_3O_2$ (295,34)
Ber.: C 69,14 H 5,80 N 14,23
Gef.: C 68,95 H 5,90 N 14,50

Beispiel 44

5-Äthyl-7,7-dimethyl-2-(2-thienyl)-6,7-dihydro-3H,5H-pyrrolo[2,3-f]benzimidazol-6-on

Hergestellt analog Beispiel 1 aus 1-Äthyl-3,3-dimethyl-6-nitro-5-(2-thenoylamido)-indolin-2-on.
Ausbeute: 67,6 % der Theorie,
Schmelzpunkt: > 290°C (Isopropanol)
$C_{17}H_{17}N_3OS$ (311,41)
Ber.: C 65,57 H 5,50 N 13,49
Gef.: C 65,48 H 5,59 N 13,60

Beispiel 45

5-Äthyl-7,7-dimethyl-2-(4-methylmercapto-phenyl)-6,7-dihydro-3H,5H-pyrrolo[2,3-f]benzimidazol-6-on

Hergestellt analog Beispiel 1 aus 1-Äthyl-3,3-dimethyl-5-(4-methylmercapto-benzoylamido)-6-nitro-indolin-2-on.
Ausbeute: 70,5 % der Theorie,
Schmelzpunkt: 293°C
$C_{20}H_{21}N_3OS$ (351,47)
Ber.: C 68,35 H 6,02 N 11,96
Gef.: C 68,21 H 6,15 N 11,96

Beispiel 46

5-Äthyl-7,7-dimethyl-2-(4-dimethylamino-phenyl)-6,7-dihydro-3H,5H-pyrrolo[2,3-f]benzimidazol-6-on

Hergestellt analog Beispiel 1 aus 1-Äthyl-3,3-dimethyl-5-(4-dimethylamino-benzoylamido)-6-nitro-indolin-2-on.
Ausbeute: 49,8 % der Theorie,

Schmelzpunkt: 287°C
$C_{21}H_{24}N_4O$ (348,45)
Ber.: C 72,39 H 6,94 N 16,08
Gef.: C 72,19 H 7,00 N 16,00

Beispiel 47

5-Äthyl-7,7-dimethyl-2-(2-methoxy-4-methylmercapto-phenyl)-6,7-dihydro-3H,5H-pyrrolo[2,3-f]benzimidazol-6-on

Hergestellt analog Beispiel 1 aus 1-Äthyl-3,3-dimethyl-5-(2-methoxy-4-methylmercapto-benzoylamido)-6-nitro-indolin-2-on.
Ausbeute: 64,5 % der Theorie,
Schmelzpunkt: 220°C
$C_{21}H_{23}N_3O_2S$ (381,50)
Ber.: C 66,12 H 6,08 N 11,01
Gef.: C 65,98 H 6,11 N 11,02

Beispiel 48

5-Äthyl-2-(2-chinolyl)-7,7-dimethyl-6,7-dihydro-3H,5H-pyrrolo[2,3-f]benzimidazol-6-on x 2,5 $H_2O$

Hergestellt analog Beispiel 1 aus 1-Äthyl-5-(2-chinolin-carbonamido)-3,3-dimethyl-6-nitro-indolin-2-on.
Ausbeute: 28,2 % der Theorie,
Schmelzpunkt: 280°C
$C_{22}H_{20}N_4O$ (356,43)
Ber.: C 65,82 H 6,28 N 13,96
Gef.: C 65,98 H 6,03 N 13,77

Beispiel 49

5-Äthyl-2-(4-amino-phenyl)-7,7-dimethyl-6,7-dihydro-3H,5H-pyrrolo[2,3-f]benzimidazol-6-on

Hergestellt analog Beispiel 1 aus 1-Äthyl-5-(4-amino-benzoyl-amido)-3,3-dimethyl-6-nitro-indolin-2-on.
Ausbeute: 55,7 % der Theorie,
Schmelzpunkt: > 295°C (Methanol/Isopropanol)
$C_{19}H_{20}N_4O$ (320,40)
Ber.: C 71,23 H 6,29 N 17,49
Gef.: C 71,18 H 6,48 N 17,37

Beispiel 50

2-(1-Acetyl-4-piperidinyl)-5-äthyl-7,7-dimethyl-6,7-dihydro-3H,5H-pyrrolo[2,3-f]benzimidazol-6-on

Hergestellt analog Beispiel 1 aus 5-(1-Acetyl-4-piperidincarbonamido)-1-äthyl-3,3-dimethyl-6-nitro-indolin-2-on.
Ausbeute: 58,8 % der Theorie,
Schmelzpunkt: 258°C
$C_{20}H_{26}N_4O_2$ (354,45)
Ber.: C 67,77 H 7,39 N 15,81
Gef.: C 67,90 H 7,52 N 16,05

Beispiel 51

5-Äthyl-2-(4-chlor-phenyl)-7,7-dimethyl-6,7-dihydro-3H,5H-pyrrolo[2,3-f]benzimidazol-6-on

Hergestellt analog Beispiel 1 aus 1-Äthyl-5-(4-chlor-benzoylamido)-3,3-dimethyl-6-nitro-indolin-2-on.
Ausbeute: 79 % der Theorie,
Schmelzpunkt: 291 °C
$C_{19}H_{18}ClN_3O$ (339,83)
Ber.: C 67,15 H 5,34 N 12,37
Gef.: C 67,30 H 5,41 N 12,37

Beispiel 52

5-Äthyl-2-(1,2,3,4-tetrahydro-4-chinolyl)-7,7-dimethyl-6,7-dihydro-3H,5H-pyrrolo[2,3-f]benzimidazol-6-on-dihydrochlorid

Hergestellt analog Beispiel 1 aus 1-Äthyl-5-(4-chinolin-carbonamido)-3,3-dimethyl-6-nitro-indolin-2-on.
Ausbeute: 45,3 % der Theorie,
Schmelzpunkt: 248-250 °C
$C_{22}H_{26}Cl_2N_4O$ (433,39)
Ber.: C 60,97 H 6,05 N 12,93
Gef.: C 61,14 H 6,23 N 13,16

Beispiel 53

5-Äthyl-2-(4-chinolyl)-7,7-dimethyl-6,7-dihydro-3H,5H-pyrrolo[2,3-f]benzimidazol-6-on

Hergestellt analog Beispiel 1 aus 1-Äthyl-5-(4-chinolin-carbonamido)-3,3-dimethyl-6-nitro-indolin-2-on.
Ausbeute: 18,3 % der Theorie,
Schmelzpunkt: 278 °C
$C_{22}H_{20}N_4O$ (356,43)
Ber.: C 74,14 H 5,66 N 15,72
Gef.: C 73,97 H 5,56 N 15,72

Beispiel 54

5-Äthyl-2-(5,6,7,8-tetrahydro-4-chinolyl)-7,7-dimethyl-6,7-dihydro-3H,5H-pyrrolo[2,3-f]benzimidazol-6-on-Hydrochlorid

Hergestellt analog Beispiel 1 aus 1-Äthyl-5-(4-chinolin-carbonamido)-3,3-dimethyl-6-nitro-indolin-2-on.
Ausbeute: 15 % der Theorie,
Schmelzpunkt: 150 °C (Zers.)
$C_{22}H_{25}ClN_4O$ (396,92)
Ber.: C 66,57 H 6,35 N 14,12
Gef.: C 66,39 H 6,45 N 13,92

Beispiel 55

5-Äthyl-7,7-dimethyl-2-(2-dimethylamino-3-pyridyl)-6,7-dihydro-3H,5H-pyrrolo[2,3-f]benzimidazol-6-on-dihydrochlorid x $H_2O$

Hergestellt analog Beispiel 1 aus 1-Äthyl-3,3-dimethyl-5-(2-dimethylamino-nicotinoylamido)-6-nitro-indolin-2-on.
Ausbeute: 62 % der Theorie,

Schmelzpunkt: ab 223°C (Zers.)
$C_{20}H_{25}Cl_2N_5O \times H_2O$ (440,37)
Ber.: C 54,55 H 6,18 N 15,90
Gef.: C 54,79 H 6,15 N 15,78

Beispiel 56

5-Äthyl-7,7-dimethyl-2-(2-morpholino-3-pyridyl)-6,7-dihydro-3H,5H-pyrrolo[2,3-f]benzimidazol-6-on-hydrochlorid × $H_2O$

Hergestellt analog Beispiel 1 aus 1-Äthyl-3,3-dimethyl-5-(2-morpholino-nicotinoylamido)-6-nitro-indolin-2-on.
Ausbeute: 28,2 % der Theorie,
Schmelzpunkt: ab 185°C (Zers.)
$C_{22}H_{26}ClN_5O_2 \times H_2O$ (445,95)
Ber.: C 59,25 H 6,33 N 15,70
Gef.: C 59,19 H 6,06 N 15,43

Beispiel 57

5-Äthyl-7,7-dimethyl-2-(2,6-dichlor-4-pyridyl)-6,7-dihydro-3H,5H-pyrrolo[2,3-f]benzimidazol-6-on

Hergestellt analog Beispiel 1 aus 1-Äthyl-5-(2,6-dichlorisonicotinoylamido)-3,3-dimethyl-6-nitro-indolin-2-on.
Ausbeute: 64,6 % der Theorie,
Schmelzpunkt: > 290°C
$C_{18}H_{16}Cl_2N_4O$ (375,27)
Ber.: C 57,61 H 4,30 N 14,63
Gef.: C 57,42 H 4,32 N 14,38

Beispiel 58

5-Äthyl-2-(2-chlor-6-morpholino-4-pyridyl)-7,7-dimethyl-6,7-dihydro-3H,5H-pyrrolo[2,3-f]benzimidazol-6-on

Hergestellt analog Beispiel 1 aus 1-Äthyl-5-(2-chlor-6-morpholino-isonicotinoylamido)-3,3-dimethyl-6-nitro-indolin-2-on.
Ausbeute: 54,3 % der Theorie,
Schmelzpunkt: > 290°C
$C_{22}H_{24}ClN_5O_2$ (425,93)
Ber.: C 62,04 H 5,68 N 16,44
Gef.: C 61,91 H 5,70 N 16,23

Beispiel 59

5-Äthyl-7,7-dimethyl-2-(5-pyrimidinyl)-6,7-dihydro-3H,5H-pyrrolo[2,3-f]benzimidazol-6-on

Hergestellt analog Beispiel 1 aus 1-Äthyl-3,3-dimethyl-5-(pyrimidin-5-carbonamido)-6-nitro-indolin-2-on.
Ausbeute: 29,7 % der Theorie,
Schmelzpunkt: > 300°C
$C_{17}H_{17}N_5O$ (307,36)
Ber.: C 66,43 H 5,58 N 22,79
Gef.: C 66,26 H 5,54 N 22,67

Beispiel 60

7,7-Dimethyl-5-n-propyl-2-(2-thienyl)-6,7-dihydro-3H,5H-pyrrolo[2,3-f]benzimidazol-6-on

Hergestellt analog Beispiel 1 aus 3,3-Dimethyl-1-n-propyl-6-nitro-5-(2-thenoylamido)-indolin-2-on.
Ausbeute: 55,5 % der Theorie,
Schmelzpunkt: > 280°C (Essigester/Isopropanol)
$C_{18}H_{19}N_3OS$ (325,43)
Ber.: C 66,43 H 5,88 N 12,91
Gef.: C 66,32 H 5,96 N 12,80

Beispiel 61

7,7-Dimethyl-2-(2-furyl)-5-n-propyl-6,7-dihydro-3H,5H-pyrrolo[2,3-f]benzimidazol-6-on

Hergestellt analog Beispiel 1 aus 3,3-Dimethyl-5-(2-furoylamido)-6-nitro-1-n-propyl-indolin-2-on.
Ausbeute: 58 % der Theorie,
Schmelzpunkt: > 290°C (Essigester/Isopropanol)
$C_{18}H_{19}N_3O_2$ (309,37)
Ber.: C 69,88 H 6,19 N 13,58
Gef. C 69,62 H 6,21 N 13,57

Beispiel 62

7,7-Dimethyl-2-(4-methylmercapto-phenyl)-5-n-propyl-6,7-dihydro-3H,5H-pyrrolo[2,3-f]benzimidazol-6-on

Hergestellt analog Beispiel 1 aus 3,3-Dimethyl-5-(4-methylmercapto-benzoylamido)-6-nitro-1-n-propyl-indolin-2-on.
Ausbeute: 29,3 % der Theorie,
Schmelzpunkt: 286°C
$C_{21}H_{23}N_3OS$ (365,49)
Ber.: C 69,01 H 6,34 N 11,50
Gef.: C 68,87 H 6,42 N 11,36

Beispiel 63

7,7-Dimethyl-5-n-pentyl-2-(2-thienyl)-6,7-dihydro-3H,5H-pyrrolo[2,3-f]benzimidazol-6-on

Hergestellt analog Beispiel 1 aus 3,3-Dimethyl-6-nitro-1-n-pentyl-5-(2-thenoylamido)-indolin-2-on.
Ausbeute: 70,3 % der Theorie,
Schmelzpunkt: 216°C
$C_{20}H_{23}N_3OS$ (353,49)
Ber.: C 67,96 H 6,56 N 11,89
Gef.: C 67,77 H 6,61 N 11,89

Beispiel 64

5-Äthyl-2-(4-cyan-phenyl)-7,7-dimethyl-6,7-dihydro-3H,5H-pyrrolo[2,3-f]benzimidazol-6-on x 0,5 $H_2O$

4 g (10,5 mMol) 1-Äthyl-5-(4-cyan-benzoylamido)-3,3-dimethyl-6-nitro-indolin-2-on werden in 100 ml Eisessig gelöst, mit 0,5 g 10 %iger Palladiumkohle versetzt und während 1 Stunde bei Raumtemperatur und 5 bar Wasserstoffdruck hydriert. Nachdem man vom Katalysator abfiltriert hat, wird das Filtrat in dem nunmehr 1-Äthyl-6-amino-5-(4-cyan-benzoyl-amino)-3,3-dimethyl-indolin-2-on vorliegt, zum Zwecke der Cyclisierung 3 Stunden auf dem Dampfbad erhitzt. Nach Abdestillieren des Lösungsmittels wird der

Rückstand in Äthylenchlorid/Äthanol = 5:1 aufgenommen, mit verdünntem Ammoniak und dann mit Wasser gewaschen, getrocknet und die Lösungsmittel abdestilliert.
Ausbeute: 1,15 g (33,9 % der Theorie),
Schmelzpunkt: > 295°C
$C_{20}H_{18}N_4O$ × 0,5 $H_2O$ (339,40)
Ber.: C 70,78 H 5,64 N 16,51
Gef.: C 70,58 H 5,60 N 16,38

Beispiel 65

5-Äthyl-2-(2-chlor-phenyl)-7,7-dimethyl-6,7-dihydro-3H,5H-pyrrolo[2,3-f]benzimidazol-6-on-hydrochlorid

a) Eine Lösung von 5,8 g (15 mMol) 1-Äthyl-5-(2-chlor-benzoylamido)-3,3-dimethyl-6-nitro-indolin-2-on in 60 ml Eisessig wird mit 0,6 g Raney-Nickel versetzt und während 4 Stunden bei 80°C und 5 bar Wasserstoffdruck hydriert. Nach Absaugen des Katalysators wird das Lösungsmittel abdestiliert, der Rückstand in Wasser gelöst und mit Ammoniak alkalisch gestellt. Der Niederschlag wird abgesaugt und mit Wasser, Essigester und schließlich mit Äther gewaschen. Es handelt sich um 1-Äthyl-6-amino-5-(2-chlor-benzoylamido)-3,3-dimethyl-indol-2-on.
Ausbeute: 1,6 g (30 % der Theorie),
Schmelzpunkt: 169°C
$C_{19}H_{20}ClN_3O_2$ (357,85)
Ber.: C 63,77 H 5,63 N 11,74 Cl 9,91
Gef.: C 63,79 H 5,62 N 11,67 Cl 9,72

b) Die unter a) erhaltene Verbindung wird in 50 ml Ätnanol und 50 ml konz. Salzsäure gelöst und 2 Tage zum Rückfluß erhitzt. Die nach dem Abkühlen ausfallenden Kristalle werden abgesaugt, mit Wasser, Aceton und schließlich mit Äther gewaschen.
Ausbeute: 1,3 g (73,5 % der Theorie),
Schmelzpunkt: 305°C (Zers.)
$C_{19}H_{19}C_{12}N_3O$ (376,29)
Ber.: C 60,65 H 5,09 N 11,17 Cl 18,84
Gef.: C 60,77 H 4,95 N 11,09 Cl 18,72

Beispiel 66

2-(2-Phenyl-vinyl)-5,7,7-trimethyl-6,7-dihydro-3H,5H-pyrrolo[2,3-f]benzimidazol-6-on

Hergestellt analog Beispiel 65 aus 5-(2-Phenylacrylamido)-6-nitro-1,3,3-trimethyl-indolin-2-on.
Ausbeute: 73 % der Theorie,
Schmelzpunkt: 243-244°C (Essigester)
$C_{20}H_{19}N_3O$ (317,39)
Ber.: C 75,69 H 6,03 N 13,24
Gef.: C 75,47 H 6,25 N 12,96

Beispiel 67

2-(2-Pyrazinyl)-5,7,7-trimethyl-6,7-dihydro-3H,5H-pyrrolo[2,3-f]benzimidazol-6-on

Hergestellt analog Beispiel 65 aus 6-Nitro-5-(2-pyrazinoylamido)-1,3,3-trimethyl-indolin-2-on.
Ausbeute: 61,6 % der Theorie,
Schmelzpunkt: > 280°C
$C_{16}H_{15}N_5O$ (293,33)
Ber.: C 65,52 H 5,15 N 23,88
Gef.: C 65,34 H 5,18 N 23,61

Beispiel 68

2-(2-Thienyl)-5,7,7-trimethyl-6,7-dihydro-3H,5H-pyrrolo[2,3-f]benzimidazol-6-on × 0,5 $H_2O$

Hergestellt analog Beispiel 65 aus 6-Nitro-5-(2-thenoylamido)-1,3,3-trimethyl-indolin-2-on.
Ausbeute: 37,8 % der Theorie,
Schmelzpunkt: ab 130°C
$C_{16}H_{15}N_3OS$ × 0,5 $H_2O$ (306,38)
Ber.: C 62,72 H 5,26 N 13,72
Gef.: C 62,93 H 5,26 N 13,62

Beispiel 69

7,7-Dimethyl-5-n-propyl-2-(2-pyrazinyl)-6,7-dihydro-3H,5H-pyrrolo[2,3-f]benzimidazol-6-on × 0,3 $H_2O$

Hergestellt analog Beispiel 65 aus 3,3-Dimethyl-6-nitro-1-n-propyl-5-(2-pyrazinoylamido)-indolin-2-on.
Ausbeute: 54,7 % der Theorie,
Schmelzpunkt: 268°C
$C_{18}H_{19}N_5O$ × 0,3 $H_2O$ (326,79)
Ber.: C 66,16 H 6,05 N 21,43
Gef.: C 66,29 H 5,93 N 21,25

Beispiel 70

5-Äthyl-7,7-dimethyl-2-(4-methyl-oxazol-5-yl)-6,7-dihydro-3H,5H-pyrrolo[2,3-f]benzimidazol-6-on

Eine Lösung von 2,6 g (7,25 mMol) 1-Äthyl-3,3-dimethyl-5-(4-methyl-5-oxazolyl-carbonamido)-6-nitro-indolin-2-on in 50 ml Eisessig wird mit 0,3 g 5 %iger Platinkohle versetzt und hydriert. Nach Beendigung der Wasserstoffaufnahme wird vom Katalysator abgesaugt und das Filtrat 3 Stunden zum Rückfluß erhitzt, das Lösungsmittel abdestilliert, der Rückstand mit Wasser versetzt, mit Ammoniak alkalisch gestellt und mit Äthylenchlorid extrahiert. Der Extrakt wird getrocknet, das Lösungsmittel abdestilliert und der Rückstand aus Essigester umkristallisiert.
Ausbeute: 1 g (44,4 % der Theorie),
Schmelzpunkt: 244°C
$C_{17}H_{18}N_4O_2$ (310,36)
Ber.: C 65,79 H 5,85 N 18,05
Gef.: C 65,60 H 5,98 N 17,98

Beispiel 71

5-Äthyl-2-(4-amino-3,5-dichlor-phenyl)-7,7-dimethyl-6,7-dihydro-3H,5H-pyrrolo[2,3-f]benzimidazol-6-on

Hergestellt analog Beispiel 70 aus 1-Äthyl-5-(4-amino-3,5-dichlor-benzoylamido)-3,3-dimethyl-6-nitro-indolin-2-on.
Ausbeute: 53 % der Theorie,
Schmelzpunkt: > 300°C (Eisessig/Äther)
$C_{19}H_{18}C_{12}N_4O$ (389,30)
Ber.: C 58,62 H 4,66 N 14,39
Gef.: C 58,49 H 4,76 N 14,60

Beispiel 72

5-Äthyl-7,7-dimethyl-2-(4-pyrimidinyl)-6,7-dihydro-3H,5H-pyrrolo[2,3-f]benzimidazol-6-on-hydrochlorid

Hergestellt analog Beispiel 70 aus 1-Äthyl-3,3-dimethyl-6-nitro-5-(pyrimidin-4-carbonamido)-indolin-2-on.
Ausbeute: 11 % der Theorie,
Schmelzpunkt: 198°C (Zers.)
$C_{17}H_{18}ClN_5O$ (343,82)
Ber.: C 59,39 H 5,28 N 20,37
Gef.: C 59,59 H 5,45 N 20,54

## Beispiel 73

5-Äthyl-7,7-dimethyl-2-(4-pyridyl)-6,7-dihydro-3H,5H-pyrrolo[2,3-f]benzimidazol-6-on

3 g (0.01 Mol) 1-Äthyl-5,6-diamino-3,3-dimethyl-indolin-2-on-dihydrochlorid werden zusammen mit 2,67 g (0,015 Mol) Isonicotinsäurechlorid-hydrochlorid in 100 ml Methylenchlorid vorgelegt und bei Raumtemperatur tropfenweise mit 5 g Triäthylamin versetzt und 1 Stunde gerührt. Nach Abdestillieren des Lösungsmittels werden zum Rückstand 50 ml Äthanol, 25 ml konz. Salzsäure und 10 ml Wasser gegeben und 18 Stunden zum Rückfluß erhitzt. Nach Abdestillieren der Lösungsmittel nimmt man den Rückstand in Äthanol auf. stellt mit Ammoniak alkalisch und gibt langsam Wasser zu. Der Niederschlag wird abgesaugt und aus Isopropanol/Diisopropyläther umkristallisiert.
Ausbeute: 1,3 g (42,5 % der Theorie),
Schmelzpunkt: 296-298°C
$C_{18}H_{18}N_4O$ (306,37)
Ber.: C 70,57 H 5,92 N 18,29
Gef.: C 70,41 H 6,00 N 18,06

## Beispiel 74

5-Äthyl-7,7-dimethyl-2-(4-nitro-phenyl)-6,7-dihydro-3H,5H-pyrrolo[2,3-f]benzimidazol-6-on

Hergestellt analog Beispiel 73 aus 4-Nitro-benzoylchlorid und 1-Äthyl-5,6-diamino-3,3-dimethyl-indolin-2-on.
Ausbeute: 23 % der Theorie,
Schmelzpunkt: > 290°C
$C_{19}H_{18}N_4O_3$ (350,38)
Ber.: C 65,13 H 5,18 N 15,99
Gef.: C 65,35 H 5,15 N 16,16

## Beispiel 75

2-(4-Pyridyl)-1,5,7,7-tetramethyl-6,7-dihydro-1H,5H-pyrrolo[2,3-f]benzimidazol-6-on

Hergestellt analog Beispiel 1 aus 6-Nitro-5-(N-methyl-isonicotinoylamido)-1,3,3-trimethyl-indolin-2-on.
Ausbeute: 42 % der Theorie,
Schmelzpunkt: 250-252°C (Essigester)
$C_{18}H_{18}N_4O$ (306,37)
Ber.: C 70,57 H 5,92 N 18,29
Gef.: C 70,54 H 6,05 N 18,26

## Beispiel 76

7,7-Dimethyl-5-n-pentyl-2-(2-pyrazinyl)-6,7-dihydro-3H,5H-pyrrolo[2,3-f]benzimidazol-6-on

Hergestellt analog Beispiel 65 aus 3,3-Dimethyl-6-nitro-1-n-pentyl-5-(2-pyrazinoylamido)-indolin-2-on.
Ausbeute: 48,8 % der Theorie,
Schmelzpunkt: 215-217°C
$C_{20}H_{23}N_5O$ (349,43)
Ber.: C 68,75 H 6,63 N 20,04
Gef.: C 68,55 H 6,68 N 20,05

Beispiel 77

7,7-Dimethyl-2-(2-furyl)-5-n-pentyl-6,7-dihydro-3H,5H-pyrrolo[2,3-f]benzimidazol-6-on

Hergestellt analog Beispiel 1 aus 3,3-Dimethyl-5-(2-furoylamido)-6-nitro-1-n-pentyl-indolin-2-on.
Ausbeute: 87 % der Theorie,
Schmelzpunkt: 152-154°C (Essigester/Diisopropyläther)
$C_{20}H_{23}N_3O_2$ (337,42)
Ber.: C 71,19 H 6,87 N 12,45
Gef.: C 70,92 H 7,02 N 12,38

Beispiel 78

5-Äthyl-7,7-dimethyl-2-[2-(4-pyridyl)-äthyl]-6,7-dihydro-3H,5H-pyrrolo[2,3-f]benzimidazol-6-on

2,92 g (0,01 Mol) 1-Äthyl-5,6-diamino-3,3-dimethyl-indolin-2-on-dihydrochlorid und 2 g (0,013 Mol) 3-(4-Pyridyl)propionsäure werden gut vermischt, mit 30 g Phosphoroxychlorid versetzt und 1 Stunde bei 100°C gerührt. Das überschüssige Phosphoroxychlorid wird dann abdestilliert, der Rückstand mit Eiswasser versetzt, mit Ammoniak alkalisch gestellt und 3 mal mit einer Mischung von Chloroform und Äthanol (4:1) extrahiert. Die Extraktionslösung wird mit Wasser gewaschen, mit Natriumsulfat getrocknet und die Lösungsmittel abdestilliert. Zur säulenchromatographischen Reinigung wird der Rückstand in Äthylenchlorid/Äthanol = 9:1 aufgenommen, auf eine Kieselgelsäule (Korngröße 40-63 $\mu$) gegeben und mit demselben Lösungsmittelgemisch eluiert. Die einheitlichen Fraktionen werden vereinigt, die Lösungsmittel abdestilliert und der Rückstand aus Essigester/Diisopropyläther umkristallisiert.
Ausbeute: 1,68 g (50 % der Theorie),
Schmelzpunkt: 139-141°C
$C_{20}H_{22}N_4O$ (334,43)
Ber.: C 71,83 H 6,63 N 16,75
Gef.: C 71,59 H 6,82 N 16,49

Beispiel 79

5-Äthyl-7,7-dimethyl-2-(3-picolyl)-6,7-dihydro-3H,5H-pyrrolo[2,3-f]benzimidazol-6-on

Hergestellt analog Beispiel 78 aus 3-Pyridyl-essigsäure und 1-Äthyl-5,6-diamino-3,3-dimethyl-indolin-2-on.
Ausbeute: 16,3 % der Theorie,
Schmelzpunkt: 194°C (Essigester)
$C_{19}H_{20}N_4O$ (320,40)
Ber.: C 71,23 H 6,29 N 17,49
Gef.: C 71,23 H 6,38 N 17,61

Beispiel 80

5-Äthyl-7,7-dimethyl-2-[2-(4-pyridyl)-vinyl]-6,7-dihydro-3H,5H-pyrrolo[2,3-f]benzimidazol-6-on

Hergestellt analog Beispiel 78 aus 3-(4-Pyridyl)-acrylsäure und 1-Äthyl-5,6-diamino-3,3-dimethyl-indolin-2-on.
Ausbeute: 23 % der Theorie,
Schmelzpunkt: 262-263°C (Essigester/Diisopropyläther))
$C_{20}H_{20}N_4O$ (332,41)
Ber.: C 72,27 H 6,06 N 16,86
Gef.: C 72,05 H 6,13 N 16,69

Beispiel 81

5-Äthyl-2-(4-chlor-7-trifluormethyl-3-chinolyl)-7,7-dimethyl-6,7-dihydro-3H,5H-pyrrolo[2,3-f]benzimidazol-6-on × 0,5 $H_2O$

Hergestellt analog Beispiel 78 aus 4-Hydroxy-7-trifluormethyl-chinolin-3-carbonsäure und 1-Äthyl-5,6-diamino-3,3-dimethyl-indolin-2-on.
Ausbeute: 18 % der Theorie,
Schmelzpunkt: 198-199°C (Essigester)
$C_{23}H_{18}ClF_3N_4O$ × 0,5 $H_2O$ (467,88)
Ber.: C 59,04 H 4,09 N 11,98
Gef.: C 59,29 H 4,15 N 12,20

Beispiel 82

5-Äthyl-7,7-dimethyl-2-(4-pyridazinyl)-6,7-dihydro-3H,5H-pyrrolo[2,3-f]benzimidazol-6-on × 0,5 $H_2O$

Hergestellt analog Beispiel 78 aus Pyridazin-4-carbonsäure und 1-Äthyl-5,6-diamino-3,3-dimethyl-indolin-2-on.
Ausbeute: 15 % der Theorie,
Schmelzpunkt: > 290°C
$C_{17}H_{17}N_5O$ × 0,5 $H_2O$ (316,36)
Ber.: C 64,54 H 5,73 N 22,14
Gef.: C 64,75 H 5,81 N 21,93

Beispiel 83

5-Äthyl-7,7-dimethyl-2-(6-methyl-3-pyridyl)-6,7-dihydro-3H,5H-pyrrolo[2,3-f]benzimidazol-6-on

Hergestellt analog Beispiel 78 aus 6-Methyl-nicotinsäure und 1-Äthyl-5,6-diamino-3,3-dimethyl-indolin-2-on.
Ausbeute: 26,3 % der Theorie,
Schmelzpunkt: 267°C
$C_{19}H_{20}N_4O$ (320,40)
Ber.: C 71,23 H 6,29 N 17,49
Gef.: C 70,98 H 6,29 N 17,23

Beispiel 84

2-(2-Chlor-6-methoxy-4-chinolyl)-5-äthyl-7,7-dimethyl-6,7-dihydro-3H,5H-pyrrolo[2,3-f]benzimidazol-6-on

Hergestellt analog Beispiel 78 aus 2-Hydroxy-6-methoxy-chi nolin-4-carbonsäure und 1-Äthyl-5,6-diamino-3,3-dimethyl-indolin-2-on.
Ausbeute: 30 % der Theorie,

Schmelzpunkt: ab 198°C (Dioxan)
$C_{23}H_{21}ClN_4O_2$ (420,91)
Ber.: C 65,63 H 5,03 N 13,31
Gef.: C 65,40 H 4,96 N 13,36

Beispiel 85

5-Äthyl-7,7-dimethyl-2-(2-phenyl-4-chinolyl)-6,7-dihydro-3H,5H-pyrrolo[2,3-f]benzimidazol-6-on × 0,5 $H_2O$

Hergestellt analog Beispiel 78 aus 2-Phenyl-chinolin-4-carbonsäure und 1-Äthyl-5,6-diamino-3,3-dimethyl-indolin-2-on.
Ausbeute: 25,5 % der Theorie,
Schmelzpunkt: ab 165°C (Toluol)
$C_{28}H_{24}N_4O$ × 0,5 $H_2O$ (441,53)
Ber.: C 76,17 H 5,71 N 12,69
Gef.: C 75,95 H 5,92 N 12,45

Beispiel 86

5-Äthyl-7,7-dimethyl-2-(4-pyridyl)-6,7-dihydro-3H,5H-pyrrolo[2,3-f]benzimidazol-6-on

0,73 g (0,06 Mol) Isonicotinsäure wird in 30 ml wasserfreiem Tetrahydrofuran gelöst, mit 0,97 g (0,06 Mol) Carbonyldiimidazol versetzt und 1 Stunde bei 30-40°C gerührt. Zu dieser Lösung tropft man eine Lösung von 1,1 g (0,05 Mol) 1-Äthyl-5,6-diamino-3,3-dimethyl-indolin-2-on in 20 ml wasserfreiem Tetrahydrofuran und rührt 3 Stunden bei Raumtemperatur. Nach Abdestillieren des Lösungsmittels versetzt man den Rückstand mit 40 ml 6 n Salzsäure und rührt 8 Stunden bei 80°C. Die Lösung wird weitgehend eingeengt, mit Ammoniak alkalisch gestellt und 2 mal mit Chloroform/Äthanol = 4:1 extrahiert. Der Extrakt wird gewaschen, mit Natriumsulfat getrocknet und die Lösungsmittel abdestilliert. Den Rückstand kristallisiert man aus Isopropanol um.
Ausbeute: 0,9 g (58,6 % der Theorie),
Schmelzpunkt: 295-297°C

Beispiel 87

5-Äthyl-2,7,7-trimethyl-6,7-dihydro-3H,5H-pyrrolo[2,3-f]benzimidazol-6-on × 0,25 $H_2O$

2,92 g (0,01 Mol) 1-Äthyl-5,6-diamino-3,3-dimethyl-indolin-2-on-dihydrochlorid werden in 20 ml Eisessig 4 Stunden lang zum Rückfluß erhitzt. Nach Abdestillieren des Lösungsmittels wird der Rückstand in 15 ml Methanol aufgenommen, mit wäßrigem Ammoniak auf pH = 8 gestellt und mit Wasser auf ein Volumen von 100 ml gebracht. Der Niederschlag wird abgesaugt, mit Wasser gewaschen, getrocknet und 1 mal aus Essigester/Diisopropyläther umkristallisiert.
Ausbeute: 1,9 g (76,7 % der Theorie),
Schmelzpunkt: 102-103°C
$C_{14}H_{17}N_3O$ × 0,25 $H_2O$ (247,81)
Ber.: C 67,81 H 7,12 N 16,96
Gef.: C 67,71 H 7,22 N 17,11

Beispiel 88

5-Äthyl-7,7-dimethyl-2-(4-pyridyl)-6,7-dihydro-3H,5H-pyrrolo[2,3-f]benzimidazol-6-on

Zu einer Lösung von 2,92 g (0,01 Mol) 1-Äthyl-5,6-diamino-3,3-dimethyl-indolin-2-on-dihydrochlorid in 20 ml Äthanol und 2 ml Eisessig gibt man 1,07 g (0,01 Mol) Pyridin-4-aldehyd. Die tiefrote Lösung wird 1 Stunde und nach Zugabe von weiteren 20 ml Äthanol noch weitere 2 Stunden unter Einleitung von

Luftsauerstoff zum Rückfluß erhitzt. Nach Abdestillieren der Lösungsmittel wird der Rückstau in Äthanol suspendiert, mit wäßrigem Ammoniak alkalisch gestellt und durch weitere Zugabe von Wasser das Reaktionsprodukt ausgefällt. Es wird abgesaugt und einmal aus Isopropanol/Diisopropyläther umkristallisiert.
Ausbeute: 1,3 g (42,5 % der Theorie),
Schmelzpunkt: 296-298°C

Beispiel 89

5-Äthyl-7,7-dimethyl-2-(2-methoxy-4-methylsulfinyl-phenyl)-6,7-dihydro-3H,5H-pyrrolo[2,3-f]benzimidazol-6-on-hydrochlorid

Eine Lösung von 1,9 g ( 5 mMol) 5-Äthyl-7,7-dimethyl-2-(2-methoxy-4-methylmercapto-phenyl)-6,7-dihydro-3H,5H-pyrrolo[2,3-f]benzimidazol-6-on in 50 ml Chloroform wird unter Rühren bei Raumtemperatur mit 1,3 g ( 6 mMol) 80 %iger m-Chlorperoxybenzoesäure versetzt und 1 Stunde nachgerührt. Die Reaktionslösung wird mit verdünnter Natronlauge und dann mit Wasser gewaschen, weitgehendst eingeengt und zur Reinigung auf eine Kieselgelsäule (Korngröße 40-63 μ) gegeben und mit Äthylenchlorid/Äthanol (20:1) eluiert. Die einheitlichen Fraktionen werden vereinigt, die Lösungsmittel abdestilliert, der ölige Rückstand in Aceton aufgenommen und mit isopropanolischer Salzsäure versetzt. Den kristallinen Niederschlag saugt man ab und wäscht mit Aceton und Äther.
Ausbeute: 1,4 g (64,5 % der Theorie),
Schmelzpunkt: 253°C (Zers.)
$C_{21}H_{24}ClN_3O_3S$ (433,97)
Ber.: C 58,12 H 5,57 N 9,68
Gef.: C 58,33 H 5,84 N 9,87

Beispiel 90

5-Äthyl-7,7-dimethyl-2-(4-methylsulfinyl-phenyl)-6,7-dihydro-3H,5H-pyrrolo[2,3-f]benzimidazol-6-on × 0,5 $H_2O$

Hergestellt analog Beispiel 89 aus 5-Äthyl-7,7-dimethyl-2-(4-methylmercapto-phenyl)-6,7-dihydro-3H,5H-pyrrolo[2,3-f]benzimidazol-6-on und m-Chlor-perbenzoesäure.
Ausbeute: 16 % der Theorie,
Schmelzpunkt: 238°C
$C_{20}H_{21}N_3O_2S$ × 0,5 $H_2O$ (376,47)
Ber.: C 63,81 H 5,89 N 11,16
Gef.: C 63,65 H 5,68 N 10,98

Beispiel 91

5-Äthyl-7,7-dimethyl-2-(2-methoxy-4-methylsulfonyl-phenyl)-6,7-dihydro-3H,5H-pyrrolo[2,3-f]benzimidazol-6-on-hydrochlorid

Eine Lösung von 1,3 g (3,4 mMol) 5-Äthyl-7,7-dimethyl-2-(2-methoxy-4-methylmercapto-phenyl)-6,7-dihydro-3H,5H-pyrrolo[2,3-f]benzimidazol-6-on in 10 ml Ameisensäure wird mit 1 ml 30 %igem Wasserstoffperoxid versetzt und 1 Stunde bei Raumtemperatur gerührt. Darauf verdünnt man mit Eiswasser, stellt mit Ammoniak alkalisch, saugt das ausgefallene Rohprodukt ab, trocknet, nimmt es in Methylenchlorid auf und bringt die Lösung zur Reinigung auf einer Kieselgelsäule (Korngröße: 40-63 μ) auf. Die mit einer Mischung aus Äthylenchlorid/Äthanol = 40:1 als Elutionsmittel gewonnenen einheitlichen Fraktionen werden vereinigt, die Lösungsmittel abdestilliert, der schmierige Rückstand in Essigester aufgenommen und mit isopropanolischer Salzsäure versetzt. Den Niederschlag saugt man ab und wäscht ihn mit Aceton und Äther.
Ausbeute: 1,5 g (97,8 % der Theorie),

Schmelzpunkt: ab 255°C (Zers.)
$C_{21}H_{24}ClN_3O_4S$ (449,95)
Ber.: C 56,06 H 5,38 N 9,34
Gef.: C 56,25 H 5,41 N 9,26

Beispiel 92

5-Äthyl-7,7-dimethyl-2-(4-methylsulfonyl-phenyl)-6,7-dihydro-3H,5H-pyrrolo[2,3-f]benzimidazol-6-on

Hergestellt analog Beispiel 91 aus 5-Äthyl-7,7-dimethyl-2-(4-methylmercapto-phenyl)-6,7-dihydro-3H,5H-pyrrolo[2,3-f]benzimidazol-6-on und Wasserstoffperoxid.
Ausbeute: 62 % der Theorie,
Schmelzpunkt: 262°C
$C_{20}H_{21}N_3O_3S$ (383,47)
Ber.: C 62,64 H 5,52 N 10,96
Gef.: C 62,56 H 5,76 N 11,14

Beispiel 93

2-(4-Acetamido-phenyl)-5-äthyl-7,7-dimethyl-6,7-dihydro-3H,5H-pyrrolo[2,3-f]benzimidazol-6-on × $H_2O$

8 g (25 mMol) 5-Äthyl-2-(4-amino-phenyl)-7,7-dimethyl-6,7-dihydro-3H,5H-pyrrolo[2,3-f]benzimidazol-6-on werden mit 10 ml Acetanhydrid versetzt und 2 Stunden bei Raumtemperatur gerührt. Der dabei erhaltene orangefarbene Brei wird mit Essigester digeriert, abgesaugt und auf einer Kieselgelsäule (Korngröße: 40-63 µ, Elutionsmittel: Äthylenchlorid/Essigester/Äthanol = 5:5:1) chromatographiert. Die einheitlichen Fraktionen werden vereinigt, die Lösungsmittel abdestilliert, der Rückstand mit Essigester aufgerührt und abgesaugt.
Ausbeute: 4 g (42,1 % der Theorie),
Schmelzpunkt: > 290°C
$C_{21}H_{22}N_4O_2$ × $H_2O$ (380,45)
Ber.: C 66,30 H 6,36 N 14,73
Gef.: C 66,47 H 6,10 N 14,71

Beispiel 94

5-Äthyl-7,7-dimethyl-2-(4-piperidinyl)-6,7-dihydro-3H,5H-pyrrolo[2,3-f]benzimidazol-6-on × 0,5 $H_2O$

1,02 g (3,3 mMol) 5-Äthyl-7,7-dimethyl-2-(4-pyridyl)-6,7-dihydro-3H,5H-pyrrolo[2,3-f]benzimidazol-6-on werden in 100 ml Eisessig gelöst, mit 0,2 g Platinoxid versetzt und im Autoklaven bei Raumtemperatur und 3 bar Wasserstoffdruck während 6 Stunden hydriert. Man saugt vom Katalysator ab, engt das Filtrat ein, löst den Rückstand in 15 ml Wasser, sättigt mit Kaliumcarbonat und extrahiert mit Chloroform. Die Chloroform-Phase wird mit gesättigter Kochsalzlösung gewaschen, mit Natriumsulfat getrocknet und das Lösungsmittel abdestil liert. Den festen Rückstand kristallisiert man 1 mal aus Essigester/Äther um.
Ausbeute: 0,8 g (74,8 % der Theorie),
Schmelzpunkt: 141-142°C
$C_{18}H_{24}N_4O$ × 0,5 $H_2O$ (321,43)
Ber.: C 67,26 H 7,84 N 17,43
Gef.: C 67,35 H 8,05 N 17,21

Beispiel 95

5-Äthyl-7,7-dimethyl-6,7-dihydro-3H,5H-pyrrolo[2,3-f]benzimidazol-6-on-hydrochlorid × 0,5 $H_2O$

Hergestellt analog Beispiel 87 aus 1-Äthyl-5,6-diamino-3,3-dimethyl-indolin-2-on und Ameisensäure.
Ausbeute: 80 % der Theorie,
Schmelzpunkt: 250-252°C
$C_{13}H_{16}ClN_3O$ × 0,5 $H_2O$ (265,75)
Ber.: C 56,83 H 6,24 N 15,29
Gef.: C 57,09 H 6,45 N 15,44

Beispiel 96

2-(4-Pyridyl)-3,5,7,7-tetramethyl-6,7-dihydro-3H,5H-pyrrolo[2,3-f]benzimidazol-6-on

Hergestellt analog Beispiel 1 aus 5-Nitro-6-(N-methyl-sonicotinoylamido)-1,3,3-trimethyl-indolin-2-on.
Ausbeute: 77 % der Theorie,
Schmelzpunkt: 218-219°C (Essigester/Diisopropyläther)
$C_{18}H_{18}N_4O$ (306,37)
Ber.: C 70,57 H 5,92 N 18,29
Gef.: C 70,40 H 5,94 N 18,32

Beispiel 97

5-Methyl-2-(4-pyridyl)-6,7-dihydro-3H,5H-pyrrolo[2,3-f]benzimidazol-6-on × 0,5 $H_2O$

Hergestellt analog Beispiel 1 aus 5-Isonicotinoylamido-1-methyl-6-nitro indolin-2-on.
Ausbeute: 65 % der Theorie,
Schmelzpunkt: > 290°C (Isopropanol)
$C_5H_{12}N_4O$ × 0,5 $H_2O$ (273,29)
Ber.: C 65,92 H 4,79 N 20,50
Gef.: C 65,74 H 4,90 N 20,59

Beispiel 98

7,7-Dimethyl-2-(4-methylmercapto-phenyl)-5-n-pentyl-6,7-dihydro-3H,5H-pyrrolo[2,3-f]benzimidazol-6-on × 0,6 $H_2O$

Hergestellt analog Beispiel 1 aus 3,3-Dimethyl-5-(4-methylmercapto-benzoylamido)-6-nitro-1-n-pentyl-indolin-2-on.
Ausbeute: 58 % der Theorie,
Schmelzpunkt: 244°C (Essigester/Isopropanol)
$C_{23}H_{27}N_3OS$ × 0,6 $H_2O$ (404,35)
Ber.: C 68,32 H 7,03 N 10,39
Gef.: C 68,11 H 7,05 N 10,27

Beispiel 99

7,7-Dimethyl-2-(4-methylsulfonyl-phenyl)-5-n-pentyl-6,7-dihydro-3H,5H-pyrrolo[2,3-f]benzimidazol-6-on × $H_2O$

Hergestellt analog Beispiel 91 aus 7,7-Dimethyl-2-(4-methyl-mercapto-phenyl)-5-n-pentyl-6,7-dihydro-3H,5H-pyrrolo[2,3-f]benzimidazol-6-on und Wasserstoffperoxid.
Ausbeute: 51,5 % der Theorie,

Schmelzpunkt: 267°C
$C_{23}H_{27}N_3O_3S \times H_2O$ (443,56)
Ber.: C 62,28 H 6,59 N 9,47
Gef.: C 62,11 H 6,39 N 9,20

Beispiel 100

5-Äthyl-7,7-dimethyl-2-(2-pyrrolyl)-6,7-dihydro-3H,5H-pyrrolo[2,3-f]benzimidazol-6-on

Hergestellt analog Beispiel 78 aus Pyrrol-2-carbonsäure und 1-Äthyl-5,6-Diamino-3,3-dimethyl-indolin-2-on.
Ausbeute: 17,5 % der Theorie,
Schmelzpunkt: > 290°C
$C_{17}H_{19}ClN_4O$ (330,82)
Ber.: C 61,72 H 5,79 N 16,94 Cl 10,72
Gef.: C 61,44 H 5,92 N 16,70 Cl 10,53

Beispiel 101

7,7-Dimethyl-5-(2-methoxy-äthyl)-2-(5-methyl-3-furyl)-6,7-dihydro-3H,5H-pyrrolo[2,3-f]benzimidazol-6-on

Hergestellt analog Beispiel 1 aus 3,3-Dimethyl-1-(2-methoxy-äthyl)-5-(5-methyl-3-furoylamido)-6-nitro-indolin-2-on.
Ausbeute: 46,4 % der Theorie,
Schmelzpunkt: 254-256°C (Essigester)
$C_{19}H_{21}N_3O_3$ (339,39)
Ber.: C 67,24 H 6,24 N 12,38
Gef.: C 67,22 H 6,35 N 12,45

Beispiel 102

5-Äthyl-7,7-dimethyl-2-(5-methyl-3-furyl)-6,7-dihydro-3H,5H-pyrrolo[2,3-f]benzimidazol-6-on

Hergestellt analog Beispiel 1 aus 1-Äthyl-3,3-dimethyl-5-(5-methyl-3-furoylamido)-6-nitro-indolin-2-on.
Ausbeute: 56,6 % der Theorie,
Schmelzpunkt: 276-278°C
$C_{18}H_{19}N_3O_2$ (309,37)
Ber.: C 69,88 H 6,19 N 13,58
Gef.: C 69,74 H 6,19 N 13,48

Beispiel 103

7,7-Dimethyl-5-isopropyl-2-(5-methyl-3-furyl)-6,7-dihydro-3H,5H-pyrrolo[2,3-f]benzimidazol-6-on

Hergestellt analog Beispiel 1 aus 3,3-Dimethyl-1-isopropyl-5-(5-methyl-3-furoylamido)-6-nitro-indolin-2-on.
Ausbeute: 69 % der Theorie,
Schmelzpunkt: > 290°C
$C_{19}H_{21}N_3O_2$ (323,39)
Ber.: C 70,57 H 6,55 N 12,99
Gef.: C 70,68 H 6,64 N 13,07

Beispiel 104

7,7-Dimethyl-2-(4-methyl-sulfinyl-phenyl)-5-n-pentyl-6,7-dihydro-3H,5H-pyrrolo[2,3-f]benzimidazol-6-on

Hergestellt analog Beispiel 89 aus 7,7-Dimethyl-2-(4-methylmercapto-phenyl)-5-n-pentyl-6,7-dihydro-3H,5H-pyrrolo[2,3-f]benzimidazol-6-on.
Ausbeute: 61 % der Theorie,
Schmelzpunkt: 235°C
$C_{23}H_{27}N_3O_2S$ (409,55)
Ber.: C 67,45 H 6,65 N 10,26 S 7,83
Gef.: C 67,43 H 6,75 N 10,12 S 7,58

Beispiel 105

7,7-Dimethyl-2-(5-methyl-3-furyl)-5-n-pentyl-6,7-dihydro-3H,5H-pyrrolo[2,3-f]benzimidazol-6-on

Hergestellt analog Beispiel 1 aus 3,3-Dimethyl-5-(5-methyl-3-furoylamido)-6-nitro-1-n-pentyl-indolin-2-on.
Ausbeute: 48,5 % der Theorie,
Schmelzpunkt: 210-212°C
$C_{21}H_{25}N_3O_2$ (351,45)
Ber.: C 71,77 H 7,17 N 11,96
Gef.: C 71,76 H 7,22 N 12,04

Beispiel 106

5-Äthyl-7,7-dimethyl-2-phenyl-6,7-dihydro-3H,5H-pyrrolo[2,3-f]benzimidazol-6-on

Hergestellt analog Beispiel 1 aus 1-Äthyl-5-benzoylamido-3,3-dimethyl-6-nitro-indolin-2-on.
Ausbeute: 63 % der Theorie,
Schmelzpunkt: 308°C (Zers.)
$C_{19}H_{19}N_3O$ (305,38)
Ber.: C 74,73 H 6,27 N 13,76
Gef.: C 74,54 H 6,30 N 13,74

Beispiel 107

5-Äthyl-7,7-dimethyl-2-n-propyl-6,7-dihydro-3H,5H-pyrrolo[2,3-f]benzimidazol-6-on

Hergestellt analog Beispiel 1 aus 1-Äthyl-5-butyrylamido-3,3-dimethyl-6-nitro-indolin-2-on.
Ausbeute: 30 % der Theorie,
Harz, Rf-Wert: 0,47 (Kieselgel, Äthylenchlorid/Äthanol = 9:1)
$C_{16}H_{21}N_3O \times 0,85\ CHCl_3$ (370,71)
Ber.: C 54,59 H 5,94 N 11,34
Gef.: C 54,44 H 6,04 N 11,25

Beispiel 108

5-Äthyl-7,7-dimethyl-2-(2-thienyl-methyl)-6,7-dihydro-3H,5H-pyrrolo[2,3-f]benzimidazol-6-on × 0,6 $H_2O$

Hergestellt analog Beispiel 1 aus 1-Äthyl-3,3-dimethyl-6-ni tro-5-(2-thienyl-acetamido)-indolin-2-on.
Ausbeute: 50,7 % der Theorie,
Schmelzpunkt: ab 115°C
$C_{18}H_{19}N_3OS \times 0,6\ H_2O$ (336,24)
Ber.: C 64,30 H 6,06 N 12,50 S 9,53
Gef.: C 64,53 H 6,27 N 12,28 S 9,32

Beispiel 109

5-Äthyl-7,7-dimethyl-2-(3-thienyl-methyl)-6,7-dihydro-3H,5H-pyrrolo[2,3-f]benzimidazol-6-on

Hergestellt analog Beispiel 1 aus 1-Äthyl-3,3-dimethyl-6-nitro-5-(3-thienyl-acetamido)-indolin-2-on.
Ausbeute: 46,2 % der Theorie,
Schmelzpunkt: ab 105°C
$C_{18}H_{19}N_3OS$ (325,44)
Ber.: C 66,43 H 5,88 N 12,91 S 9,85
Gef.: C 66,27 H 6,00 N 12,74 S 9,68

Beispiel 110

7,7-Dimethyl-5-(2-hydroxy-äthyl)-2-(3-pyridyl)-6,7-dihydro-3H,5H-pyrrolo[2,3-f]benzimidazol-6-on

Hergestellt analog Beispiel 10 aus 7,7-Dimethyl-5-(2-methoxyäthyl)-2-(3-pyridyl)-6,7-dihydro-3H,5H-pyrrolo[2,3-f]benzimidazol-6-on und Bortribromid.
Ausbeute: 52,7 % der Theorie,
Schmelzpunkt: 285-286°C
$C_{18}H_{18}N_4O_2$ (322,37)
Ber.: C 67,07 H 5,63 N 17,38
Gef.: C 66,80 H 5,76 N 17,29

Beispiel 111

5-n-Butyl-7,7-dimethyl-2-(2-thienyl-methyl)-6,7-dihydro-3H,5H-pyrrolo[2,3-f]benzimidazol-6-on

Hergestellt analog Beispiel 1 aus 1-n-Butyl-3,3-dimethyl-6-nitro-5-(2-thienyl-acetamido)-indolin-2-on.
Ausbeute: 13,7 % der Theorie,
Schmelzpunkt: ab 70°C
$C_{20}H_{23}N_3OS$ (353,48)
Ber.: C 67,96 H 6,56 N 11,89 S 9,07
Gef.: C 67,83 H 6,78 N 12,04 S 8,92

Beispiel 112

5-n-Butyl-7,7-dimethyl-2-(3-thienyl-methyl)-6,7-dihydro-3H,5H-pyrrolo[2,3-f]benzimidazol-6-on

Hergestellt analog Beispiel 1 aus 1-n-Butyl-3,3-dimethyl-6-nitro-5-(3-thienyl-acetamido)-indolin-2-on.
Ausbeute: 53,0 % der Theorie,
Schmelzpunkt: 118-120°C
$C_{20}H_{23}N_3OS$ (353,48)
Ber.: C 67,96 H 6,56 N 11,89 S 9,07
Gef.: C 67,76 H 6,62 N 11,98 S 8,90

Beispiel 113

5-n-Butyl-7,7-dimethyl-2-(2-thienyl)-6,7-dihydro-3H,5H-pyrrolo[2,3-f]benzimidazol-6-on

Hergestellt analog Beispiel 1 aus 1-n-Butyl-3,3-dimethyl-6-nitro-5-(2-thenoylamido)-indolin-2-on.
Ausbeute: 94,5 % der Theorie,
Schmelzpunkt: 271 °C (Essigester/Äthanol)
$C_{19}H_{21}N_3OS$ (339,45)
Ber.: C 67,23 H 6,24 N 12,38
Gef.: C 67,18 H 6,26 N 12,32

Beispiel 114

7,7-Dimethyl-2-(4-methylsulfinyl-phenyl)-5-n-propyl-6,7-dihydro-3H,5H-pyrrolo[2,3-f]benzimidazol-6-on

Hergestellt analog Beispiel 89 aus 7,7-Dimethyl-2-(4-methylmercapto-phenyl)-5-n-propyl-6,7-dihydro-3H,5H-pyrrolo[2,3-f]benzimidazol-6-on und m-Chlor-perbenzoesäure.
Ausbeute: 65,6 % der Theorie,
Schmelzpunkt: 242 °C
$C_{21}H_{23}N_3O_2S \times 0,2\ H_2O$ (385,09)
Ber.: C 65,50 H 6,12 N 10,91 S 8,33
Gef.: C 65,66 H 6,12 N 10,83 S 8,15

Beispiel 115

7,7-Dimethyl-2-(4-methylsulfonyl-phenyl)-5-n-propyl-6,7-dihydro-3H,5H-pyrrolo[2,3-f]benzimidazol-6-on

Hergestellt analog Beispiel 91 aus 7,7-Dimethyl-2-(4-methylmercapto-phenyl)-5-n-propyl-6,7-dihydro-3H,5H-pyrrolo[2,3-f]benzimidazol-6-on und Wasserstoffperoxid.
Ausbeute: 55,4 % der Theorie,
Schmelzpunkt: 273-275 °C
$C_{21}H_{23}N_3O_3S$ (397,49)
Ber.: C 63,46 H 5,83 N 10,57 S 8,07
Gef.: C 63,30 H 5,91 N 10,37 S 8,11

Beispiel I

Dragées mit 5 mg 5-Äthyl-7,7-dimethyl-2-(4-pyridyl)-6,7-dihydro-3H,5H-pyrrolo-[2,3-f]benzimidazol-6-on

1 Drageékern enthält:

| | |
|---|---|
| Wirksubstanz | 5,0 mg |
| Milchzucker | 25,2 mg |
| Maisstärke | 18,0 mg |
| Cellulose, mikrokristallin | 10,0 mg |
| Polyvinylpyrrolidon | 1,0 mg |
| Magnesiumstearat | 0,8 mg |
| | 60,0 mg |

Herstellverfahren:

Der Wirkstoff, Milchzucker, Maisstärke, Cellulose und Polyvinylpyrrolidon werden gemischt und mit Wasser granuliert. Das Granulat wird bei einer Temperatur von 45° C getrocknet und die angegebene Menge Magnesiumstearat zugemischt. Auf einer Tablettiermaschine werden bikonvexe Kerne mit einem Durchmesser von 5 mm hergestellt.

Dragierung:

Die Kerne werden mit einer 15 %igen Polyvinylpyrrolidon-Lösung überzogen und mit Dragiersuspension bis zu einem Gewicht von 80 mg fertigdragiert.

Beispiel II

Tabletten mit Teilkerbe zu 50 mg 5-Äthyl-7,7-dimethyl-2-(4-pyridyl)-6,7-dihydro-3H,5H-pyrrolo-[2,3-f]-benzimidazol-6-on

Zusammensetzung:

1 Tablette enthält:

| | |
|---|---|
| Wirksubstanz | 50,0 mg |
| Milchzucker | 40,0 mg |
| Maisstärke | 17,0 mg |
| Polyvinylpyrrolidon | 2,0 mg |
| Magnesiumstearat | 1,0 mg |
| | 110,0 mg |

Herstellungsverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50° C wird erneut gesiebt (1,5 mm-Maschenweite) und das Schmiermittel zugemischt.

Die preßfertige Mischung wird zu Tabletten mit Teilkerbe verarbeitet.

Beispiel III

Suppositorien mit 100 mg 5-Äthyl-7,7-dimethyl-2-(4-pyridyl)-6,7-dihydro-3H,5H-pyrrolo-[2,3-f]benzimidazol-6-on

1 Zäpfchen enthält:

| | |
|---|---|
| Wirksubstanz | 100,0 mg |
| Suppositorienmassen (Hartfett) | 1600,0 mg |
| | 1700,0 mg |

Herstellungsverfahren:

Die feinpulverisierte Wirksubstanz wird in die geschmolzene und auf 40° C abgekühlte Zäpfchenmasse einhomogenisiert. Man kühlt auf 37° C ab und gießt die Masse in leicht vorgekühlte Formen aus. Zäpfchengewicht: 1,7 g.

Beispiel IV

Ampullen zu 10 mg 5-Äthyl-7,7-dimethyl-2-(4-pyridyl)-6,7-dihydro-3H,5H-pyrrolo-[2,3-f]benzimidazol-6-on

Zusammensetzung: Wirksubstanz 10,0 mg
0,1 n HCl 0,34 ml
Aqua bidest. ad 1,0 ml

Herstellung:

Die Substanz wird in Wasser unter Zusatz von Salzsäure gelöst, unter aseptischen Bedingungen durch einen Membranfilter filtriert und anschließend in braune, gereinigte und sterilisierte 2 ml-Injektionsfläschchen abgefüllt.

**Ansprüche**

1. Neue Pyrrolo-benzimidazole der Formel

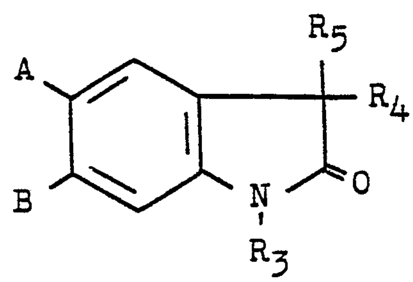

(I)

in der
A und B zusammen mit den beiden dazwischenliegenden Kohlenstoffatomen eine Imidazogruppe der Formel

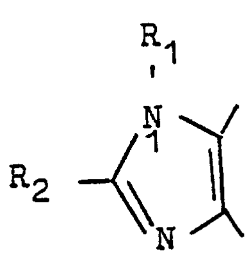

oder

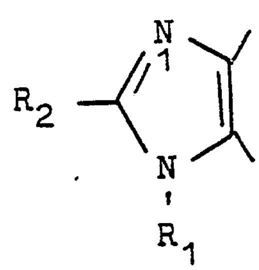

darstellt, wobei
$R_1$ ein Wasserstoffatom oder eine gegebenenfalls durch eine Phenylgruppe substituierte Alkylgruppe mit 1 bis 3 Kohlenstoffatomen und
$R_2$ ein Wasserstoffatom, eine gegebenenfalls durch eine Phenyl-, Pyridyl-oder Thienylgruppe substituierte Alkylgruppe, eine Vinylengruppe, die endständig durch eine Phenyl-oder Pyridylgruppe substituiert ist, eine

gegebenenfalls durch ein Halogenatom, eine Hydroxy-, Alkoxy-, Mercapto-, Alkylmercapto-, Alkylsulfinyl-, Alkylsulfonyl-, Cyano-, Amino-, Alkylamino-, Dialkylamino-, Alkanoylamino-oder Ni trogruppe substituierte Phenylgruppe, wobei eine Hydroxyphenylgruppe zusätzlich durch eine oder zwei Alkylgruppen mit jeweils 1 bis 4 Kohlenstoffatomen oder eine Aminophenylgruppe durch ein oder zwei Halogenatome substituiert sein kann, eine durch Halogenatome, Hydroxy-, Alkoxy-, Mercapto-, Alkylmercapto-, Alkylsulfinyl-oder Alkylsulfonylgruppen disubstituierte Phenylgruppe, wobei die Substituenten des Phenylkerns gleich oder verschieden sein können, einen über ein Kohlenstoffatom gebundenen 5-oder 6-gliedrigen heteroaromatischen Ring, welcher ein Sauerstoff-, Schwefel-oder Stickstoffatom, zwei oder drei Stickstoffatome oder ein Stickstoffatom und ein Sauerstoff-oder Schwefelatom enthält, an den zusätzlich über zwei benachbarte Kohlenstoffatome eine oder zwei 1,4-Butadienylgruppen gebunden sein können, wobei in diesem Falle auch die Bindung über einen ankondensierten Phenylring erfolgen kann und im Falle der Pyridine diese zusätzlich durch eine Amino-, Alkylamino-, Dialkylamino-, Alkanoylamino-oder Morpholinogruppe oder durch zwei Halogenatome oder durch ein Halogenatom und eine Amino-oder Morpholinogruppe substituiert und im Falle der Chinoline zusätzlich durch ein Halogenatom, eine Alkoxy-oder Phenylgruppe monooder disubstituiert, wobei die Substituenten gleich oder verschieden sein können, sein können, einen über einen Kohlenstoffatom gebundenen 5-bis 7-gliedrigen gesättigten Imino-, N-Alkyl-imino-oder N-Alkanoyl-iminoalkylenring, eine 5,6,7,8-Tetrahydro-chinolylgruppe oder eine gegebenenfalls am N-Atom durch eine Alkanoylgruppe substituierte 1,2,3,4-Tetrahydro-chinolylgruppe, wobei alle bei der Definition des Restes $R_2$ vorstehend erwähnten Ringe durch eine Alkylgruppe substituiert sein können und der Alkylteil aller vorstehend erwähnten Gruppen, sofern nichts anderes erwähnt wird, jeweils 1 bis 3 Kohlenstoffatome und der Alkanoylteil zwei oder drei Kohlenstoffatome enthalten kann,

$R_3$ eine gegebenenfalls durch eine Phenylgruppe substituierte Alkylgruppe, eine Alkylgruppe mit 4 bis 6 Kohlenstoffatomen oder eine in 2-oder 3-Position durch eine Hydroxy-, Alkoxy-oder Pyridiniumgruppe substituierte Alkylgruppe, wobei der Alkylteil jeweils 1 bis 3 Kohlenstoffatome enthalten kann,

$R_4$ und $R_5$, die gleich oder verschieden sein können, Wasserstoffatome, Alkylgruppen mit 1 bis 3 Kohlenstoffatomen oder Cycloalkylgruppen mit 3 bis 7 Kohlenstoffatomen bedeuten, und deren Säureadditionssalze.

2. Neue Pyrrolo-benzimidazole der Formel I gemäß Anspruch 1, in der

$R_2$ bis $R_5$ wie im Anspruch 1 definiert sind und

$R_1$ ein Wasserstoffatom darstellt, deren 3H-Tautomere und deren Säureadditionssalze.

3. Neue Pyrrolo-benzimidazole der allgemeinen Formel I gemäß Anspruch 1, in der

$R_1$ ein Wasserstoffatom,

$R_2$ ein Wasserstoffatom, eine Alkylgruppe mit 1 bis 3 Kohlenstoffatomen, eine durch eine Phenyl-, Pyridyl- oder Thienylgruppe substituierte Alkylgruppe mit 1 oder 2 Kohlenstoffatomen im Alkylteil, eine endständig durch eine Phenyl-oder Pyridylgruppe substituierte Vinylengruppe, eine gegebenenfalls durch eine Methyl-, Amino-, Methylamino-, Dimethylamino-oder Morpholinogruppe substituierte Pyridylgruppe, eine durch 2 Halogenatome oder durch ein Halogenatom und eine Morpholinogruppe substituierte Pyridylgruppe, eine gegebenenfalls durch eine Methylgruppe substituierte Furyl-, Thienyl-, Pyrrolyl-, Oxazolyl-, Pyrimidinyl-, Pyrazinyl-, Pyridazinyl-, Chinolyl-oder Acridinylgruppe, eine durch eine Phenylgruppe oder durch ein Halogenatom und eine Methoxy-oder Trifluormethylgruppe substituierte Chinolylgruppe, eine gegebenenfalls am N-Atom durch eine Acetylgruppe sub stituierte Piperidinyl-oder 1,2,3,4-Tetrahydro-chinolylgruppe oder eine 5,6,7,8-Tetrahydro-chinolylgruppe, eine gegebenenfalls durch ein Halogenatom, eine Hydroxy-, Methoxy-, Methylmercapto-, Methylsulfinyl-, Methylsulfonyl-, Nitro-, Amino-, Methylamino-, Dimethylamino-, Acetylamino-oder Cyanogruppe substituierte Phenylgruppe, eine durch ein Halogenatom, eine Methoxy-, Methylmercapto-, Methylsulfinyl-oder Methylsulfonylgruppe disubstituierte Phenylgruppe, wobei die Substituenten gleich oder verschieden sein können, eine Hydroxy-di-tert.Butylphenyl-oder Amino-dihalogenphenylgruppe,

$R_3$ eine Alkylgruppe mit 1 bis 5 Kohlenstoffatomen, eine Benzyl-, 2-Hydroxy-äthyl-oder 2-Methoxyäthylgruppe,

$R_4$ und $R_5$, die gleich oder verschieden sein können, Wasserstoffatome oder Methylgruppen bedeuten, deren 3H-Tautomere und deren Säureadditionssalze.

4. Neue Pyrrolo-benzimidazole der Formel gemäß Anspruch 1, in der

$R_1$ ein Wasserstoffatom,

$R_2$ eine durch eine Cyano-, Dimethylamino-, Methoxy-, Methylmercapto-, Methylsulfinyl-oder Methylsulfonylgruppe substituierte Phenylgruppe, eine gegebenenfalls durch eine Methyl-, Amino-, Methylamino-, Dimethylamino-oder Morpholinogruppe substituierte Pyridylgruppe, eine durch 2 Halogenatome oder durch ein Halogenatom und eine Morpholinogruppe substituierte Pyridylgruppe, eine gegebenenfalls durch eine Methylgruppe substituierte Furyl-, Thienyl-, Pyrrolyl-, Oxazolyl-, Pyrimidinyl-, Pyrazinyl-, Pyridazinyl-,

Chinolyl-oder Acridinylgruppe,
$R_3$ eine Alkylgruppe mit 1 bis 5 Kohlenstoffatomen,
$R_4$ ein Wasserstoffatom oder eine Methylgruppe und
$R_5$ eine Methylgruppe bedeuten, deren 3H-Tautomere und deren Säureadditionssalze.

5. Neue Pyrrolo-benzimidazole der Formel I gemäß Anspruch 1, in der
$R_1$ ein Wasserstoffatom,
$R_2$ eine in 4-Stellung durch eine Cyano-, Dimethylamino-, Methylsulfinyl-oder Methylsulfonylgruppe substituierte Phenylgruppe, eine Furyl-2-, Thienyl-2-, Pyridyl-, 4-Pyrimidinyl-oder 4-Methyl-oxazol-5-yl-gruppe,
$R_3$ eine Alkylgruppe mit 1 bis 3 Kohlenstoffatomen,
$R_4$ und $R_5$ jeweils eine Methylgruppe bedeuten, deren 3H-Tautomere und deren Säureadditionssalze.

6. 5-Äthyl-7,7-dimethyl-2-(4-pyridyl)-6,7-dihydro-3H,5H-pyrrolo-[2,3-f]benzimidazol-6-on, dessen 3H-Tautomere und dessen Säureadditionssalze.

7. Physiologisch verträgliche Säureadditionssalze der Verbindungen gemäß den Ansprüchen 1 bis 6 mit anorganischen oder organischen Säuren.

8. Arzneimittel, enthaltend eine Verbindung der allgemeinen Formel I gemäß den Ansprüchen 1 bis 6 oder dessen physiologisch verträgliches Säureadditionssalz gemäß Anspruch 7 neben einem oder mehreren inerten Trägerstoffen und/oder Verdünnungsmitteln.

9. Verwendung einer Verbindung gemäß den Ansprüchen 1 bis 6 oder deren physiologisch verträgliche Säureadditionssalze gemäß Anspruch 7 zur Herstellung eines Arzneimittels gemäß Anspruch 8.

10. Verfahren zur Herstellung eines Arzneimittels gemäß Anspruch 8, dadurch gekennzeichnet, daß auf nichtchemischem Wege eine Verbindung gemäß den Ansprüchen 1 bis 6 oder dessen physiologisch verträgliches Säureadditionssalz gemäß Anspruch 7 in einen oder mehrere inerte Trägerstoffe und/oder Verdünnungsmittel eingearbeitet wird.

11. Verfahren zur Herstellung von neuen Pyrrolo-benzimidazolen gemäß den Ansprüchen 1 bis 7, dadurch gekennzeichnet, daß

a) eine Verbindung der Formel

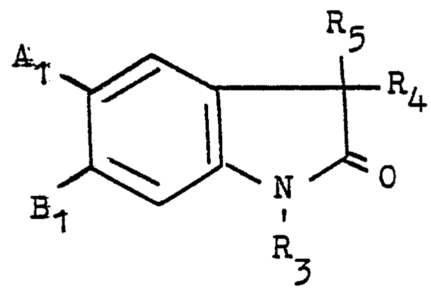

(II)

in der R
$R_3$ bis $R_5$ wie in den Ansprüchen 1 bis 6 definiert sind
einer der Reste $A_1$ oder $B_1$ eine $R_1NH$-Gruppe, wobei $R_1$ wie in den Ansprüchen 1 bis 6 definiert ist, und der andere der Reste $A_1$ oder $B_1$ eine

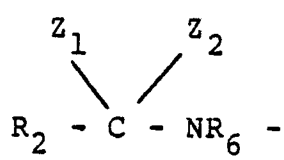

Gruppe bedeuten, wobei
$R_2$ wie in den Ansprüchen 1 bis 6 definiert ist und $R_6$ ein Wasserstoffatom oder auch, falls $R_1$ ein Wasserstoffatom darstellt, eine gegebenenfalls durch eine Phenylgruppe substituierte Alkylgruppe mit 1 bis 3 Kohlenstoffatomen,
$Z_1$ und $Z_2$, die gleich oder verschieden sein können, nukleophile Austrittsgruppen wie gegebenenfalls substituierte Amino-, Alkoxy-, Phenylalkoxy-, Phenoxy-, Alkylmercapto-, Phenylalkylmercapto-oder Phenylthiogruppen oder
$Z_1$ und $Z_2$ zusammen ein Sauerstoff-oder Schwefelatom, eine gegebenenfalls durch eine Alkyl-oder Phenylalkylgruppe substituierte Iminogruppe, wobei die vorstehend erwähnten Alkylteile jeweils 1 bis 3

Kohlenstoffatome enthalten können, oder eine Alkylendioxigruppe mit 2 oder 3 Kohlenstoffatomen bedeuten, cyclisiert wird oder

b) eine Verbindung der Formel

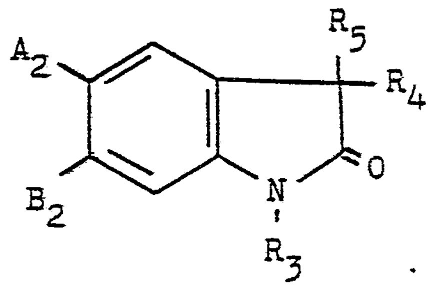

(III)

in der

$R_3$, $R_4$ und $R_5$ wie in den Ansprüchen 1 bis 6 definiert sind,

einer der Reste $A_2$ oder $B_2$ eine $R_1$NH-Gruppe, wobei $R_1$ wie in den Ansprüchen 1 bis 6 definiert ist, und der andere der Reste $A_2$ oder $B_2$ eine $NH_2$-Gruppe bedeuten, mit einem Aldehyd der Formel

$R_2$ -CHO ,(IV)

in der

$R_2$ wie in den Ansprüchen 1 bis 6 definiert ist, umgesetzt und anschließend eine so erhaltene Verbindung oxidiert wird und

gewünschtenfalls eine so erhaltene Verbindung der Formel I, in der $R_1$ ein Wasserstoffatom darstellt und/oder $R_2$ eine Hydroxy-oder Mercaptogruppe enthält, mittels Alkylierung in die entsprechende Alkylverbindung übergeführt wird und/oder

eine so erhaltene Verbindung der Formel I, in der $R_2$ eine Aminogruppe enthält, mittels Alkanoylierung in die entsprechende Alkanoylverbindung übergeführt wird und/oder

eine so erhaltene Verbindung der Formel I, in der $R_2$ eine endständig durch eine Phenyl-oder Pyridylgruppe substituierte Vinylengruppe darstellt, mittels katalytischer Hydrierung in eine entsprechende Verbindung der Formel I, in der $R_2$ eine endständig durch eine Phenyl-oder Pyridylgruppe substituierte Ethylengruppe darstellt, übergeführt wird und/oder

eine so erhaltene Verbindung der Formel I, in der $R_1$ eine Benzylgruppe darstellt, mittels katalytischer Hydrierung in eine entsprechende Verbindung der Formel I, in der $R_1$ ein Wasserstoff darstellt, übergeführt wird und/oder

eine so erhaltene Verbindung der Formel I, in der $R_2$ ein Pyridinring darstellt, mittels katalytischer Hydrierung in eine entsprechende Verbindung der Formel I, in der $R_2$ eine Piperidinylgruppe darstellt, übergeführt wird und/oder

eine so erhaltene Verbindung der Formel I, in der $R_2$ eine Alkoxyphenyl-oder Dialkoxyphenylgruppe darstellt, mittels Etherspaltung in eine entsprechende Verbindung der Formel I, in der $R_2$ eine Hydroxyphenyl-oder Dihydroxyphenylgruppe darstellt, übergeführt wird und/oder

eine so erhaltene Verbindung der Formel I, in der $R_2$ eine Alkylmercaptophenyl-oder Dialkylmercaptophenylgruppe dar stellt, mittels Oxidation in eine entsprechende Verbindung der Formel I, in der $R_2$ eine Alkylsulfinyl-, Alkylsulfonyl-, Dialkylsulfinyl-oder Dialkylsulfonyl-phenylgruppe darstellt, übergeführt wird und/oder

eine so erhaltene Verbindung der allgemeinen Formel I in ihre Säureadditionssalze, insbesondere in ihre physiologisch verträglichen Salze mit anorganischen oder organischen Säuren, übergeführt wird.

Europäisches Patentamt

# EUROPÄISCHER RECHERCHENBERICHT

Nummer der Anmeldung

EP 87 11 6512

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 161 632 (BOEHRINGER)<br>* Ansprüche 1,6,7 *<br>--- | 1,8 | C 07 D 487/04<br>A 61 K 31/415//<br>(C 07 D 487/04<br>C 07 D 235:00<br>C 07 D 209:00 ) |
| A | EP-A-0 186 010 (BOEHRINGER)<br>* Ansprüche 1,9,10 *<br>--- | 1,8 | |
| D,A | EP-A-0 189 103 (BOEHRINGER)<br>* Ansprüche 1,9,10 *<br>----- | 1,8 | |
| | | | RECHERCHIERTE SACHGEBIETE (Int. Cl.4) |
| | | | C 07 D 487/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 10-02-1988 | ALFARO I. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)